(19) Europäisches Patentamt
European Patent Office
Office européen des brevets

(11) EP 2 827 829 B1

(12) EUROPÄISCHE PATENTSCHRIFT

(45) Veröffentlichungstag und Bekanntmachung des Hinweises auf die Patenterteilung:
**08.02.2017 Patentblatt 2017/06**

(21) Anmeldenummer: **12794351.2**

(22) Anmeldetag: **03.12.2012**

(51) Int Cl.:
***A61K 8/26*** (2006.01) ***A61K 8/34*** (2006.01)
***A61K 8/39*** (2006.01) ***A61K 8/44*** (2006.01)
***A61K 8/73*** (2006.01) ***A61K 8/06*** (2006.01)
***A61Q 15/00*** (2006.01) ***A61K 8/362*** (2006.01)
***A61K 8/37*** (2006.01) ***A61K 8/42*** (2006.01)

(86) Internationale Anmeldenummer:
**PCT/EP2012/074251**

(87) Internationale Veröffentlichungsnummer:
**WO 2013/092186 (27.06.2013 Gazette 2013/26)**

(54) **PEG-FREIE ANTITRANSPIRANT-ÖL-IN-WASSER-EMULSIONEN MIT VERBESSERTER HAPTIK**

PEG-FREE ANTIPERSPIRANT OIL-IN-WATER EMULSIONS HAVING IMPROVED FEEL

ÉMULSIONS HUILE DANS L’EAU D’ANTITRANSPIRANT SANS PEG PRÉSENTANT DES PROPRIÉTÉS HAPTIQUES AMÉLIORÉES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2011 DE 102011089340**

(43) Veröffentlichungstag der Anmeldung:
**28.01.2015 Patentblatt 2015/05**

(73) Patentinhaber: **Henkel AG & Co. KGaA**
**40589 Düsseldorf (DE)**

(72) Erfinder:
- **BANOWSKI, Bernhard**
  **40597 Düsseldorf (DE)**
- **CLAAS, Marcus**
  **40723 Hilden (DE)**

(56) Entgegenhaltungen:
**EP-A1- 2 191 868**

- **"Further surfactant launches from in-cosmetics 2010", FOCUS ON SURFACTANTS, ELSEVIER ENGINEERING INFORMATION, INC, US, Bd. 2010, Nr. 7, 1. Juli 2010 (2010-07-01) , Seiten 2-3, XP027119143, ISSN: 1351-4210 [gefunden am 2010-07-01]**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 827 829 B1

**Beschreibung**

Die vorliegende Anmeldung betrifft schweißhemmende Zusammensetzungen in Form einer lotionsähnlichen Öl-in-Wasser-Emulsion mit einem Polyethylenoxid-freien Emulgatorsystem, die insbesondere zur Applikation mit einem Roll-on-Applikator geeignet sind und eine hohe Lagerstabilität und ein nicht-fettendes Hautgefühl aufweisen.

Es gibt zahlreiche Möglichkeiten, kosmetische Zusammensetzungen zur Haut- und Körperpflege auf die Haut aufzutragen. Cremes, Salben und Lotionen werden üblicherweise aus einem Tiegel, einer Tube oder einem Pumpspender entnommen und mit der Hand aufgetragen und verrieben. Formstabile Stiftmassen werden aus einem Stiftspender heraus über die Haut gestrichen, bis eine wirksame Menge aufgetragen ist. Auch Gele und Cremes können mit stiftähnlichen Dispensern, die mit einer Dispenseroberfläche über die Haut gestrichen werden, aufgetragen werden. Insbesondere für schweißhemmende und/oder desodorierende Zusammensetzungen für den Achselbereich wurden zahlreiche verschiedene Applikationsformen entwickelt, neben den bereits genannten vor allem die treibgashaltigen und treibgasfreien Sprays und die Roll-on-Zusammensetzungen. Bei letzteren wird eine leicht verdickte Flüssigkeit aus einem Vorratsbehälter über eine drehbar gelagerte Kugel durch Rollen über die Haut appliziert. Zahlreiche kosmetische Wirkstoffe, darunter die schweißhemmenden Aluminiumsalze, sind wasserlöslich und ihre Freisetzung auf der Haut könnte durch Öl- und Fettbestandteile des Kosmetikums verzögert werden. Als rein wässrige Lösung wäre das Produkt allerdings kaum zu dosieren und damit für den Verbraucher nicht akzeptabel. Durch leichtes Andicken jedoch lässt sich eine solche Zusammensetzung bequem mit einem Roll-on-Applikator verwenden. Häufig werden polymere Verdickungsmittel eingesetzt. Nachteilig hierbei ist, dass die meisten polymeren Verdickungsmittel in den erforderlichen Konzentrationen ein sehr klebriges Hautgefühl erzeugen. Darüber hinaus weisen viele dieser Verdicker keine zusätzlichen kosmetischen Pflegeeffekte auf. Eine vorteilhafte Alternative hierzu stellen Emulsionen mit einem geringen Öl- und Fettgehalt dar. Die Emulsionsbildung führt bereits ohne oder mit nur geringen Mengen an Polymerverdicker zu einem Anstieg der Viskosität. Der Öl- und Fettanteil der Emulsion entfaltet darüber hinaus eine hautpflegende Wirkung.

Emulsionen sind, im Gegensatz zu Mikroemulsionen, thermodynamisch instabil. Die thermodynamisch stabilen Mikroemulsionen können meist nur durch einen relativ hohen Emulgatorgehalt stabilisiert werden. Ein hoher Gehalt an Emulgatoren kann jedoch im ungünstigsten Fall hautreizend wirken und wird daher möglichst vermieden. Außerdem bilden sich Mikroemulsionen häufig nur in einem sehr engen Mischungsbereich der einzelnen Komponenten. Bei kosmetischen Zusammensetzungen mit mehreren Bestandteilen kann es daher entwicklungstechnisch mitunter sehr schwierig sein, geeignete Mikroemulsionsbereiche einzustellen. Emulsionen sind für eine gewisse Zeit stabil, weil die Koaleszenz der dispergierten Tröpfchen kinetisch gehemmt ist. Diese kinetische Hemmung kann aufgehoben werden durch Lagerung bei hohen Temperaturen (relevant insbesondere für die Produktion und Vermarktung in warmen Ländern) oder bei Lagerung unter größeren Temperaturschwankungen (z. B. in unzureichend klimatisierten Verkaufsräumen, beim Transport über weitere Strecken). Auch die hohe Salzkonzentration in Antitranspirant-Zusammensetzungen, bedingt durch die relativ hohe Konzentration an schweißhemmenden Wirkstoffen, kann die Emulsionsdestabilisierung begünstigen (z. B. durch Aussalzeffekte).

Typische Antitranspirant-Roll-on-Emulsionen werden mit Polyethylenglycold-haltigen Emulgatoren stabilisiert, insbesondere mit Kombinationen aus einem Emulgator mit niedrigem HLB-Wert, wie Steareth-2, und einem Emulgator mit hohem HLB-Wert, wie Steareth-20 oder Steareth-21. Die überwiegende Anzahl der im Markt befindlichen Deodorant- oder Antitranspirant-Roll-on-Formulierungen mit Aluminiumsalzen (Aluminiumchlorhydrate; Aluminiumzirconiumchlorhydrate) in Form von Öl-in-Wasser-Emulsionen enthalten ethoxylierte (=Polyethylenglycol-haltige, PEG-haltige) Fettalkohole oder Fettsäuren als Emulgatoren und propoxylierte (=Polypropylenglycol-haltige, PPG-haltige) Öle (meist Ether) als Coemulgatoren. Wie in anderen Kosmetikbereichen, wird auch im Deo/Antitranspirant-Sektor nach Ersatzmöglichkeiten für EO/PO-Addukte (EO = Ethylenoxid, PO = Propylenoxid) gesucht.

Überraschend wurde gefunden, dass sich unter Verwendung von mindestens einer oberflächenaktiven Verbindung mit einem HLB-Wert im Bereich von 9 bis 15, ausgewählt aus den Partialestern eines Polyglycerins, das 3, 4 oder 5 Glycerin-Einheiten umfasst, mit einer linearen, gesättigten Alkansäure mit 8 bis 22 C-Atomen und mit einer organischen Genusssäure, in Kombination mit mindestens einem N-Acyl-L-glutaminsäure-Natriumsalz, weiterhin mindestens einem Strukturanten, ausgewählt aus linearen, gesättigten 1-Alkanolen mit 14 bis 22 C-Atomen und Glycerylmono- und -diestern von linearen oder verzweigten, gesättigten oder ungesättigten Carbonsäuren mit 8 bis 22 C-Atomen, und weiterhin einem verhältnismäßig geringen Ölanteil stabile Öl-in-Wasser-Emulsionen herstellen lassen, die eine hohe Toleranz gegenüber Aluminiumsalzen und den damit verbundenen niedrigen pH-Werten im Bereich von 4 bis 5 aufweisen. Als Ölkomponente können verschiedene polare oder unpolare, bei Raumtemperatur flüssige Öle eingesetzt werden. Auswahlkriterium für das Öl kann hier z.B. die Auswaschbarkeit aus Textilien sein. Die für eine komfortable Applikation mittels eines Roll On-Applikators notwendige Viskosität kann mittels verschiedener Hydrogelbildner, insbesondere mittels eines Biopolymers, eingestellt werden.

Die neuartigen Antitranspirant-Emulsionen sind frei von EO- oder PO-Addukten und weisen eine verbesserte Haptik, insbesondere eine geringere Klebrigkeit, auf. In Abklatschtests auf dunklen Textilien in der Achselhöhle zeigen die neuartigen Antitranspirant-Emulsionen geringere oder gleiche Rückstände wie konventionelle Roll On-Emulsionen

auf der Basis von Steareth-2/Steareth-21.

Ein erster Gegenstand der vorliegenden Anmeldung sind daher schweißhemmende Zusammensetzungen in Form einer Öl-in-Wasser-Emulsion, die keine Mikroemulsionen sind, enthaltend

a) mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in dem Mittel beziehen, und zusätzlich dazu

b) in einer Gesamtmenge von 0,1 - 2 Gew.-%, bevorzugt 0,3 - 1,5 Gew.-%, besonders bevorzugt 0,5 - 1,1 Gew.-%, außerordentlich bevorzugt 0,6 - 0,8 Gew.-%, mindestens eine oberflächenaktive Verbindung mit einem HLB-Wert im Bereich von 9 bis 15, ausgewählt aus den Partialestern eines Polyglycerins, das 3, 4 oder 5 Glycerin-Einheiten umfasst, mit einer linearen oder verzweigten, gesättigten oder ungesättigten Carbonsäure mit 8 bis 22 C-Atomen und mit einer organischen Genusssäure,
und zusätzlich dazu

c) in einer Gesamtmenge von 0,1 - 2,0 Gew.-%, bevorzugt 0,3 - 1,2 Gew.-%, besonders bevorzugt 0,6 - 1,1 Gew.-%, außerordentlich bevorzugt 0,8 - 1,0 Gew.-%, mindestens ein N-Acyl-L-glutaminsäure-Natriumsalz der Formel (GLUT-1)

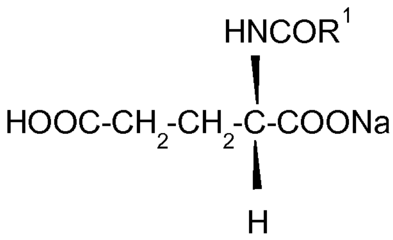

(GLUT-1),

worin $R^1CO$ eine lineare oder verzweigte, gesättigte oder ungesättigte Acylgruppe mit 6 bis 22 C-Atomen, bevorzugt mit 8 bis 18 C-Atomen, darstellt; und zusätzlich dazu

d) mindestens ein unter Normalbedingungen flüssiges kosmetisches Öl, das kein Riechstoff und kein ätherisches Öl ist, in einer Gesamtmenge von 0,5 - 10 Gew.-%, bevorzugt 1 - 8 Gew.-%, besonders bevorzugt 1,5 - 6 Gew.-%, außerordentlich bevorzugt 2 - 4,5 Gew.-%, und zusätzlich dazu

e) mindestens ein Hydrogel bildendes Polymer in einer Gesamtmenge von 0,08 - 1 Gew.-%, bevorzugt 0,1 - 0,8 Gew.-%, besonders bevorzugt 0,15 - 0,6 Gew.-%, außerordentlich bevorzugt 0,2 - 0,4 Gew.-%, und zusätzlich dazu

f) in einer Gesamtmenge von 0,05 bis 4 Gew.-%, bevorzugt 0,5 - 3,5 Gew.-%, besonders bevorzugt 1 - 3 Gew.-% und außerordentlich bevorzugt 1,5 - 2,5 Gew.-%, mindestens einen Strukturanten, ausgewählt aus linearen, gesättigten 1-Alkanolen mit 14 bis 22 C-Atomen, Glycerylmono- und -diestern von linearen oder verzweigten, gesättigten oder ungesättigten Carbonsäuren mit 8 bis 22 C-Atomen, linearen, gesättigten 1-Alkancarbonsäuren mit 14 bis 22 C-Atomen, Mono- und Diestern von Ethylenglycol mit linearen gesättigten und ungesättigten Fettsäuren mit 12 - 30, insbesondere 14 - 22 Kohlenstoffatomen, den Mono-, Di-, Tri- und Tetraestern von Pentaerythrit mit linearen gesättigten und ungesättigten Fettsäuren mit 12 - 30, insbesondere 14 - 22 Kohlenstoffatomen, sowie Mischungen dieser Strukturanten, und zusätzlich dazu

g) Wasser in einer Gesamtmenge von 30 - 90 Gew.-%, bevorzugt 40 - 80 Gew.-%, besonders bevorzugt 60 - 78 Gew.-%, außerordentlich bevorzugt 65 - 73 Gew.-%,

wobei sich die Gew.-%-Angaben jeweils auf das Gesamtgewicht der Zusammensetzung beziehen. Bevorzugte erfindungsgemäße Zusammensetzungen enthalten Polyethylenglycol-haltige und Polypropylenglycol-haltige Verbindungen in einer Gesamtmenge von 0 bis maximal 0,3 Gew.-%, bevorzugt von 0 bis maximal 0,2 Gew.-%, besonders bevorzugt von 0 bis maximal 0,1 Gew.-%, jeweils bezogen auf das Gesamtgewicht der Zusammensetzung.

Antitranspirant-Wirkstoffe

Die erfindungsgemäßen Zusammensetzungen enthalten als Antitranspirant-Wirkstoff mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in der Zusammensetzung beziehen.

Die schweißhemmenden Aluminiumsalze sind bevorzugt ausgewählt aus den wasserlöslichen adstringierenden anorganischen und organischen Salzen von Aluminium und Aluminium-Zirconium-Mischungen. Alumosilicate und Zeolithe zählen erfindungsgemäß nicht zu den Antitranspirant-Wirkstoffen. Erfindungsgemäß wird unter Wasserlöslichkeit eine Löslichkeit von wenigstens 3 Gew.-% bei 20 °C verstanden, das heißt, dass Mengen von wenigstens 3 g des Antitranspirant-Wirkstoffs in 97 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel $[Al_2(OH)_5Cl \cdot 1\text{-}6\ H_2O]_n$, bevorzugt $[Al_2(OH)_5Cl \cdot 2\text{-}3\ H_2O]_n$, das in nicht-aktivierter oder in aktivierter (depolymerisierter) Form vorliegen kann, sowie Aluminiumchlorhydrat mit der allgemeinen Formel $[Al_2(OH)_4Cl_2 \cdot 1\text{-}6\ H_2O]_n$, bevorzugt $[Al_2(OH)_4Cl_2 \cdot 2\text{-}3\ H_2O]_n$, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorhydrex-Propylenglykol (PG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG, Aluminium-PG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirconium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat ($KAl(SO_4)_2 \cdot 12\ H_2O$, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Aluminiumsalzen von Lipoaminosäuren, Aluminiumsulfat, Aluminiumlactat, Aluminiumchlorhydroxyallantoinat und Natrium-Aluminium-Chlorhydroxylactat.

Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart.

Weitere bevorzugte schweißhemmende Wirkstoffe sind basische Calcium-Aluminiumsalze, wie sie z. B. in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, wie sie z. B. in US 4017599 offenbart sind, die mit Salzen von Aminosäuren, insbesondere mit Alkali- und Erdalkaliglycinaten, gepuffert sind.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Bevorzugte schweißhemmende Aluminium-Zirconium-Salze weisen ein molares Metall-zu-Chlorid-Verhältnis von 0,9 - 1,5, bevorzugt 0,9 - 1,3, besonders bevorzugt 0,9 - 1,1, auf.

Erfindungsgemäß besonders bevorzugte Zirconium-freie Aluminiumsalze weisen ein molares Metall-zu-Chlorid-Verhältnis von 1,9 - 2,1 auf. Erfindungsgemäß besonders bevorzugte Zirconium-freie Aluminiumsesquichlorohydrate weisen ein molares Metall-zu-Chlorid-Verhältnis von 1,5:1-1,8:1 auf.

Bevorzugte Aluminiumzirconiumchlorohydrate haben im allgemeinen die empirische Formel $Al_nZr(OH)_{[3n+4-m(n+1)]}(Cl)_{[m(n+1)]}$ mit n = 2,0 - 10,0, bevorzugt 3,0 - 8,0, m = 0,77 - 1,11 (entsprechend einem molaren Metall (Al+Zr)-zu-Chlorid-Verhältnis von 1,3 - 0,9), bevorzugt m = 0,91 - 1,11 (entsprechend M:Cl = 1,1 - 0,9), und besonders bevorzugt m = 1,00 - 1,11 (entsprechend M:Cl = 1,0 - 0,9), weiterhin sehr bevorzugt m = 1,02 - 1,11 (entsprechend M:Cl = 0,98 - 0,9) sowie sehr bevorzugt m = 1,04 - 1,11 (entsprechend M:Cl = 0,96 - 0,9). Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und

der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten.

Bevorzugte schweißhemmende Salze sind Aluminium-Zirconiumtetrachlorohydrate (Al:Zr = 2-6; M:Cl = 0.9-1.3), insbesondere Salze mit einem molaren Metall-zu-Chlorid-Verhältnis von 0,9 - 1,1, bevorzugt 0,9 - 1,0.

Weiterhin erfindungsgemäß bevorzugt sind Aluminiumzirconiumchlorohydrat-Glycin-Salze, die mit Betain ($(CH_3)_3N^+-CH_2-COO^-$) stabilisiert sind. Besonders bevorzugte entsprechende Verbindungen weisen ein molares Gesamt-(Betain + Glycin)/Zr-Verhältnis von (0,1 - 3,0) : 1, bevorzugt (0,7 - 1,5): 1 und ein molares Verhältnis von Betain zu Glycin von mindestens 0,001 : 1 auf. Entsprechende Verbindungen sind beispielsweise offenbart in US 7105691.

Eine erste obligatorische Komponente des erfindungsgemäßen Emulgatorsystems ist eine oberflächenaktive Verbindung mit einem HLB-Wert im Bereich von 9 bis 15, die ausgewählt ist aus den Partialestern eines Polyglycerins, das 3, 4 oder 5 Glycerin-Einheiten umfasst, mit einer linearen oder verzweigten, gesättigten oder ungesättigten Carbonsäure mit 8 bis 22 C-Atomen und mit einer organischen Genusssäure, und die in einer Gesamtmenge von 0,1 - 2 Gew.-%, bevorzugt 0,3 - 1,5 Gew.-%, besonders bevorzugt 0,5 - 1,1 Gew.-%, außerordentlich bevorzugt 0,6 - 0,8 Gew.-%, enthalten ist, jeweils bezogen auf das Gesamtgewicht der Zusammensetzung.

Unter organischen Genusssäuren versteht man im Lebensmittelbereich und im Sinne der vorliegenden Anmeldung die folgenden organischen Säuren, die z. B. aufgrund ihres Sauergeschmacks, ihrer Pufferwirkung oder Komplexbildung bei der Herstellung und Zubereitung von Lebensmitteln verwendet werden dürfen oder natürlich in Lebensmitteln enthalten sind: Citronensäure, Adipinsäure, Hydroxybernsteinsäure, Bernsteinsäure, Milchsäure und Weinsäure. Eine erfindungsgemäß bevorzugte organische Genusssäure, die mit dem Polyglycerin mit 3, 4 oder 5 Glycerin-Einheiten verestert ist, ist Citronensäure.

Erfindungsgemäß bevorzugte Zusammensetzungen sind daher dadurch gekennzeichnet, dass die mindestens eine oberflächenaktive Verbindung b) mit einem HLB-Wert im Bereich von 9 bis 15 ausgewählt ist aus den Partialestern eines Polyglycerins, das 3, 4 oder 5 Glycerin-Einheiten umfasst, mit einer linearen, gesättigten Alkansäure mit 8 bis 22 C-Atomen und mit Citronensäure.

Besonders vorteilhafte Polyglycerinester-Emulgatoren mit 3, 4 oder 5 Glycerin-Einheiten b) mit einem HLB-Wert im Bereich von 9 bis 15 sind solche, die außer mit mindestens einer Genusssäure, bevorzugt mit Citronensäure, weiterhin mit einer linearen oder verzweigten, gesättigten oder ungesättigten Carbonsäure mit 8 bis 22 C-Atomen verestert sind, die bevorzugt ausgewählt ist aus 2-Ethylhexansäure, n-Octansäure, n-Decansäure, n-Dodecansäure, n-Tetradecansäure, n-Hexadecansäure (Palmitinsäure), n-Octadecansäure (Stearinsäure), Isostearinsäure, 12-Hydroxystearinsäure, Ölsäure, Linolsäure, Linolensäure, n-Eicosansäure und n-Docosansäure, jeweils mit endständiger Säuregruppe (das heißt, mit der Carboxylgruppe in alpha-Position), wobei die Veresterung mit Stearinsäure besonders bevorzugt ist.

Weitere erfindungsgemäß bevorzugte Zusammensetzungen sind daher dadurch gekennzeichnet, dass die mindestens eine oberflächenaktive Verbindung b) mit einem HLB-Wert im Bereich von 9 bis 15 ausgewählt ist aus den Partialestern eines Polyglycerins, das 3, 4 oder 5 Glycerin-Einheiten umfasst, mit Citronensäure und mit einer linearen oder verzweigten, gesättigten oder ungesättigten Carbonsäure mit 8 bis 22 C-Atomen verestert sind, die bevorzugt ausgewählt ist aus 2-Ethylhexansäure, n-Octansäure, n-Decansäure, n-Dodecansäure, n-Tetradecansäure, n-Hexadecansäure (Palmitinsäure), n-Octadecansäure (Stearinsäure), Isostearinsäure, 12-Hydroxystearinsäure, Ölsäure, Linolsäure, Linolensäure, n-Eicosansäure und n-Docosansäure, jeweils mit endständiger Säuregruppe (das heißt, mit der Carboxylgruppe in alpha-Position), wobei die Veresterung mit Stearinsäure besonders bevorzugt ist.

Weitere besonders vorteilhafte Polyglycerinester-Emulgatoren b) mit einem HLB-Wert im Bereich von 9 bis 15, die wie vorstehend verestert sind, weisen 3 Glycerin-Einheiten auf.

Weitere erfindungsgemäß bevorzugte Zusammensetzungen sind daher dadurch gekennzeichnet, dass die mindestens eine oberflächenaktive Verbindung b) mit einem HLB-Wert im Bereich von 9 bis 15 ausgewählt ist aus den Partialestern eines Polyglycerins, das 3 Glycerin-Einheiten umfasst, mit Citronensäure und mit einer linearen oder verzweigten, gesättigten oder ungesättigten Carbonsäure mit 8 bis 22 C-Atomen, die ausgewählt ist aus 2-Ethylhexansäure, n-Octansäure, n-Decansäure, n-Dodecansäure, n-Tetradecansäure, n-Hexadecansäure (Palmitinsäure), n-Octadecansäure (Stearinsäure), Isostearinsäure, 12-Hydroxystearinsäure, Ölsäure, Linolsäure, Linolensäure, n-Eicosansäure und n-Docosansäure, jeweils mit endständiger Säuregruppe (das heißt, mit der Carboxylgruppe in alpha-Position), wobei die Veresterung mit Stearinsäure besonders bevorzugt ist.

Der Polyglycerinester-Emulgator b) weist einen HLB-Wert im Bereich von 9 - 15 auf. Für die Viskosität und die Temperaturstabilität der erfindungsgemäßen Emulsionen bei 40°C hat es sich als besonders vorteilhaft erwiesen, wenn der Polyglycerinester-Emulgator b) einen HLB-Wert im Bereich von 10 - 14, besonders bevorzugt 11 - 12,5, aufweist.

Um einen hydrophilen Polyglycerinester-Emulgator, der wie vorstehend dargestellt verestert ist, zu erhalten, dürfen nicht alle OH-Gruppen des Polyglycerins, bevorzugt des Triglycerins, mit einer linearen oder verzweigten, gesättigten oder ungesättigten Carbonsäure mit 8 bis 22 C-Atomen oder mit Citronensäure verestert sein. Entsprechende erfindungsgemäß geeignete Polyglycerinester-Emulgatoren sind kommerziell erhältlich. Besonders bevorzugt ist die mindestens eine oberflächenaktive Verbindung b) mit einem HLB-Wert im Bereich von 9 bis 15 ausgewählt ist aus den

Partialestern eines Polyglycerins, das 3 Glycerin-Einheiten umfasst, mit Citronensäure und mit Stearinsäure, die die INCI-Bezeichnung Polyglyceryl-3 Dicitrate/Stearate tragen. Ein solches Handelsprodukt ist zum Beispiel Tego Care PSC 3 von Evonik.

Weitere erfindungsgemäß bevorzugte Zusammensetzungen sind daher dadurch gekennzeichnet, dass die mindestens eine oberflächenaktive Verbindung b) mit einem HLB-Wert im Bereich von 9 bis 15 ausgewählt ist aus den Partialestern eines Polyglycerins, das 3 Glycerin-Einheiten umfasst, mit Citronensäure und mit Stearinsäure, die die INCI-Bezeichnung Polyglyceryl-3 Dicitrate/Stearate bzw. Polyglyceryl-3 Stearate/Dicitrate tragen.

Die Ermittlung des HLB-Wertes erfolgt erfindungsgemäß nach Griffin gemäß der Formel:

$$HLB = 20 * (M_h / M),$$

wobei $M_h$ die Molmasse des hydrophilen Anteils eines Moleküls ist und M die Molmasse des gesamten Moleküls. Es ergibt sich damit eine Skala von 0 bis 20.

Zweite obligatorische Komponente des erfindungsgemäßen Emulgatorsystems ist in einer Gesamtmenge von 0,1 - 2,0 Gew.-%, bevorzugt 0,3 - 1,2 Gew.-%, besonders bevorzugt 0,6 - 1,1 Gew.-%, außerordentlich bevorzugt 0,8 - 1,0 Gew.-%, mindestens ein N-Acyl-L-glutaminsäure-Natriumsalz der Formel (GLUT-1)

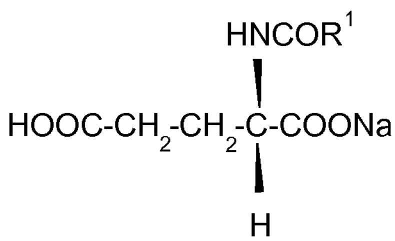

(GLUT-1),

worin $R^1CO$ eine lineare oder verzweigte, gesättigte oder ungesättigte Acylgruppe mit 6 bis 22 C-Atomen, bevorzugt mit 8 bis 18 C-Atomen, darstellt.

Erfindungsgemäß bevorzugte Zusammensetzungen enthalten mindestens ein N-Acyl-L-glutaminsäure-Natriumsalz der Formel (GLUT-1), das ausgewählt ist aus Verbindungen, worin $R^1CO$ eine n-Hexanoyl-, n-Octanoyl-, n-Decanoyl-, n-Lauroyl-, Myristoyl-, Palmitoyl-, Stearoyl-, Arachidoyl-, Behenoyl- oder Cocoyl-Gruppe, bevorzugt eine Stearoyl-Gruppe, darstellt, jeweils mit endständiger Carbonylgruppe. Außerordentlich bevorzugt ist N-Stearoylmononatrium-L-glutamat. Dieser Emulgator ist beispielsweise als Handelsprodukt Eumulgin SG von BASF erhältlich.

Für die besonders bevorzugte Darreichungsform der erfindungsgemäßen Zusammensetzungen als Roll-on ist eine nicht zu hohe Viskosität nötig. Bevorzugte erfindungsgemäße Zusammensetzungen sind daher gekennzeichnet durch eine dynamische Viskosität im Bereich von 300 - 10000 mPas, bevorzugt 800 - 7500 mPas, besonders bevorzugt 1000 - 5000 mPas, gemessen mit einem Brookfield-Viskosimeter, Spindel RV 4, 20 $s^{-1}$, ohne Helipath, bei 20 °C Umgebungstemperatur und 20 °C Probentemperatur.

Sowohl die resultierende Viskosität der Zusammensetzung als auch ihre Temperaturstabilität bei 40°C sind unter anderem abhängig vom Gewichtsverhältnis des Polyglycerinesters b) zum N-Acyl-L-glutaminsäure-Natriumsalz der Formel (GLUT-1), jeweils bezogen auf deren Gesamtgewichte.

Für Roll-ons besonders geeignete Viskositäten wie auch eine hervorragende Temperaturstabilität bei 40°C werden erzielt, wenn das Gewichtsverhältnis von Gesamtgewicht an Polyglycerinester b) zu Gesamtgewicht an N-Acyl-L-glutaminsäure-Natriumsalz der Formel (GLUT-1) im Bereich von 0,5 bis 1,0, bevorzugt 0,6 bis 0,8, liegt. Bevorzugte erfindungsgemäße Zusammensetzungen sind daher gekennzeichnet durch ein Gewichtsverhältnis von Gesamtgewicht an Polyglycerinester b) zu Gesamtgewicht an N-Acyl-L-glutaminsäure-Natriumsalz der Formel (GLUT-1) im Bereich von 0,5 bis 1,0, bevorzugt 0,6 bis 0,8.

Als weitere obligatorische Komponente enthalten die erfindungsgemäßen Zusammensetzungen mindestens ein unter Normalbedingungen flüssiges kosmetisches Öl, das kein Riechstoff und kein ätherisches Öl ist, in einer Gesamtmenge von 0,5 - 10 Gew.-%, bevorzugt 1 - 8 Gew.-%, besonders bevorzugt 1,5 - 6 Gew.-%, außerordentlich bevorzugt 2 - 4,5 Gew.-%, jeweils bezogen auf das Gewicht der Zusammensetzung.

Das kosmetische Öl ist unter Normalbedingungen flüssig. Die unter Normalbedingungen flüssigen kosmetischen Öle sind mit Wasser nicht mischbar. Unter ätherischen Ölen werden erfindungsgemäß Gemische aus flüchtigen Komponenten verstanden, die durch Wasserdampfdestillation aus pflanzlichen Rohstoffen hergestellt werden, wie z. B. Citrusöle. Sofern in der vorliegenden Anmeldung von einem kosmetischen Öl die Rede ist, handelt es sich hierbei immer

um ein kosmetisches Öl, das kein Riechstoff und kein ätherisches Öl ist, unter Normalbedingungen flüssig und mit Wasser nicht mischbar ist.

Bei den kosmetischen Ölen unterscheidet man flüchtige und nicht-flüchtige Öle. Unter nicht-flüchtigen Ölen versteht man solche Öle, die bei 20 °C und einem Umgebungsdruck von 1013 hPa einen Dampfdruck von weniger als 2,66 Pa (0,02 mm Hg) aufweisen. Unter flüchtigen Ölen versteht man solche Öle, die bei 20 °C und einem Umgebungsdruck von 1013 hPa einen Dampfdruck von 2,66 Pa - 40000 Pa (0,02 mm - 300 mm Hg), bevorzugt 10 - 12000 Pa (0,1 - 90 mm Hg), besonders bevorzugt 13 - 3000 Pa, außerordentlich bevorzugt 15 - 500 Pa, aufweisen.

Flüchtige kosmetische Öle sind üblicherweise unter cyclischen Siliconölen mit der INCI-Bezeichnung Cyclomethicone ausgewählt. Unter der INCI-Bezeichnung Cyclomethicone werden insbesondere Cyclotrisiloxan (Hexamethylcyclotrisiloxan), Cyclotetrasiloxan (Octamethylcyclotetrasiloxan), Cyclopentasiloxan (Decamethylcyclopentasiloxan) und Cyclohexasiloxan (Dodecamethylcyclohexasiloxan) verstanden. Diese Öle weisen bei 20°C einen Dampfdruck von ca. 13 - 15 Pa auf.

Cyclomethicone sind im Stand der Technik als gut geeignete Öle für kosmetische Zusammensetzungen, insbesondere für deodorierende Zusammensetzungen bekannt. Aufgrund ihrer Persistenz in der Umwelt kann es aber erfindungsgemäß bevorzugt sein, auf den Einsatz von Cyclomethiconen zu verzichten. In einer speziell bevorzugten Ausführungsform enthalten die erfindungsgemäßen und erfindungsgemäß verwendeten Zusammensetzungen 0 bis weniger als 1 Gew.-%, bevorzugt maximal 0,1 Gew.-%, Cyclomethicone, bezogen auf das Gewicht der Zusammensetzung.

Ein erfindungsgemäß bevorzugter Cyclomethicone-Ersatzstoff ist eine Mischung aus $C_{13}$-$C_{16}$-Isoparaffinen, $C_{12}$ - $C_{14}$-Isoparaffinen und $C_{13}$ - $C_{15}$-Alkanen, deren Viskosität bei 25°C im Bereich von 2 - 6 mPas liegt und die einen Dampfdruck bei 20°C im Bereich von 10 - 150 Pa, bevorzugt 100 - 150 Pa, aufweist. Eine solche Mischung ist z. B. unter der Bezeichnung SiClone SR-5 von der Firma Presperse Inc. erhältlich.

Weitere bevorzugte flüchtige Siliconöle sind ausgewählt aus flüchtigen linearen Siliconölen, insbesondere flüchtigen linearen Siliconölen mit 2 - 10 Siloxaneinheiten, wie Hexamethyldisiloxan ($L_2$), Octamethyltrisiloxan ($L_3$), Decamethyltetrasiloxan ($L_4$), wie sie z. B. in den Handelsprodukten DC 2-1184, Dow Corning® 200 (0,65 cSt) und Dow Corning® 200 (1,5 cSt) von Dow Corning enthalten sind, und niedermolekulares Phenyl Trimethicone mit einem Dampfdruck bei 20°C von etwa 2000 Pa, wie es z. B. von GE Bayer Silicones/Momentive unter dem Namen Baysilone Fluid PD 5 erhältlich ist.

Bevorzugte erfindungsgemäße Antitranspirant-Zusammensetzungen enthalten wegen des trockeneren Hautgefühls und der schnelleren Wirkstofffreisetzung mindestens ein flüchtiges Siliconöl, das cyclisch oder linear sein kann.

Weitere bevorzugte erfindungsgemäße Zusammensetzungen enthalten wegen des trockeneren Hautgefühls und der schnelleren Freisetzung des Antitranspirant-Wirkstoffs mindestens ein flüchtiges Nichtsiliconöl. Bevorzugte flüchtige Nichtsiliconöle sind ausgewählt aus $C_8$-$C_{16}$-1soparaffinen, insbesondere aus Isononan, Isodecan, Isoundecan, Isododecan, Isotridecan, Isotetradecan, Isopentadecan und Isohexadecan, sowie Mischungen hiervon. Bevorzugt sind $C_{10}$-$C_{13}$-Isoparaffin-Mischungen, insbesondere solche mit einem Dampfdruck bei 20°C von 10 - 400 Pa, bevorzugt 13 - 100 Pa.

Dieses mindestens eine $C_8$-$C_{16}$-Isoparaffin ist bevorzugt in einer Gesamtmenge von 0,5 - 10 Gew.-%, bevorzugt 1 - 8 Gew.-%, besonders bevorzugt 1,5 - 6 Gew.-%, außerordentlich bevorzugt 2 - 4,5 Gew.-%, enthalten, jeweils bezogen auf das Gesamtgewicht der Zusammensetzung. Weitere erfindungsgemäß bevorzugte Zusammensetzungen enthalten mindestens ein nichtflüchtiges kosmetisches Öl, ausgewählt aus nichtflüchtigen Siliconölen und nichtflüchtigen Nichtsiliconölen. Rückstände von in der Zusammensetzung unlöslichen Bestandteilen, wie Talkum oder Silica, aber auch die auf der Haut angetrockneten Antitranspirantwirkstoffe (= schweißhemmende Aluminiumsalze), können erfolgreich mit einem nichtflüchtigen Öl maskiert werden. Außerdem können mit einem Gemisch aus verschiedenen Ölen, insbesondere aus nichtflüchtigem und flüchtigem Öl, Parameter wie Hautgefühl, Sichtbarkeit des Rückstands und Stabilität der erfindungsgemäßen Zusammensetzung feinreguliert und besser an die Bedürfnisse der Verbraucher angepasst werden.

Erfindungsgemäß bevorzugte Mittel sind dadurch gekennzeichnet, dass das kosmetische Öl, das kein Riechstoff und kein ätherisches Öl ist, mindestens ein flüchtiges Öl mit einem Dampfdruck von 10 - 3000 Pa bei 20°C, das kein Riechstoff und kein ätherisches Öl ist, in einer Gesamtmenge von 10 - 100 Gew.-%, besonders bevorzugt 30 - 80 Gew.-%, jeweils bezogen auf das Gesamtgewicht der kosmetischen Öle, umfasst.

Selbstverständlich ist es ebenfalls möglich, erfindungsgemäße Mittel mit einem geringen Anteil an flüchtigen Ölen - das heißt, mit 0,5 - 15 Gew.-% an flüchtigen Ölen, bezogen auf das Gesamtgewicht des Mittels - oder sogar ohne flüchtige Öle zu formulieren.

Erfindungsgemäß besonders bevorzugte Öle sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Hierzu sei angemerkt, dass einige Ester von linearen oder verzweigten $C_1$-$C_{22}$-Alkanolen oder $C_{14}$-$C_{22}$-Alkenolen und einige Triester des Glycerins mit linearen oder verzweigten $C_2$-$C_{22}$-Carbonsäuren, die gesättigt oder ungesättigt sein können, unter Normalbedingungen fest sind, wie

beispielsweise Cetylstearat oder Glycerintristearat (= Stearin). Diese unter Normalbedingungen festen Ester stellen erfindungsgemäß keine kosmetischen Öle dar, da sie nicht die Bedingung "unter Normalbedingungen flüssig" erfüllen. Die Zuordnung, ob ein derartiger Ester unter Normalbedingungen flüssig oder fest ist, liegt im Rahmen des Allgemeinwissens des Fachmanns. Bevorzugt sind Ester der linearen oder verzweigten gesättigten Fettalkohole mit 2 - 5 Kohlenstoffatomen mit linearen oder verzweigten gesättigten oder ungesättigten Fettsäuren mit 3 - 18 Kohlenstoffatomen, die hydroxyliert sein können. Bevorzugte Beispiele hierfür sind Isopropylpalmitat, Isopropylstearat, Isopropylmyristat, 2-Hexyldecylstearat, 2-Hexyldecyllaurat, Isodecylneopentanoat, Isononylisononanoat, 2-Ethylhexylpalmitat und 2-Ethylhexylstearat. Ebenfalls bevorzugt sind Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat, Ethylenglycoldipalmitat, n-Hexyllaurat, n-Decyloleat, Oleyloleat, Oleylerucat, Erucyloleat, $C_{12}$-$C_{15}$-Alkyllactat und Di-$C_{12}$-$C_{13}$-Alkylmalat sowie die Benzoesäureester von linearen oder verzweigten $C_{8-22}$-Alkanolen. Besonders bevorzugt sind Benzoesäure-$C_{12}$-$C_{15}$-Alkylester, z. B. erhältlich als Handelsprodukt Finsolv® TN ($C_{12}$-$C_{15}$-Alkylbenzoat), sowie Benzoesäureisostearylester, z. B. erhältlich als Finsolv® SB, 2-Ethylhexylbenzoat, z. B. erhältlich als Finsolv® EB, und Benzoesäure-2-octyldodecylester, z. B. erhältlich als Finsolv® BOD.

Weitere erfindungsgemäß bevorzugte Ölkomponenten sind ausgewählt aus den $C_8$-$C_{22}$-Fettalkoholestern einwertiger oder mehrwertiger $C_2$-$C_7$-Hydroxycarbonsäuren, insbesondere die Ester der Glycolsäure, Milchsäure, Äpfelsäure, Weinsäure, Citronensäure und Salicylsäure. Solche Ester auf Basis von linearen $C_{14/15}$-Alkanolen, z. B. $C_{12}$-$C_{15}$-Alkyllactat, und von in 2-Position verzweigten $C_{12/13}$-Alkanolen sind unter dem Handelsnamen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Als besonders vorteilhaft, z. B. für die Wirkstofffreisetzung, hat sich der Einsatz von Isopropylestern von $C_{12}$-$C_{18}$-Carbonsäuren, insbesondere der Einsatz von Isopropylmyristat, und besonders bevorzugt Mischungen von Isopropylmyristat mit $C_{10}$-$C_{13}$-Isoparaffin-Mischungen, letztere bevorzugt mit einem Dampfdruck bei 20°C von 10 - 400 Pa, erwiesen.

Erfindungsgemäß bevorzugte Mittel enthalten mindestens einen Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, in einer Gesamtmenge von 0,5 - 10 Gew.-%, bevorzugt 1 - 8 Gew.-%, besonders bevorzugt 1,5 - 6 Gew.-%, außerordentlich bevorzugt 2 - 4,5 Gew.-%, jeweils bezogen auf das Gewicht der gesamten Zusammensetzung.

Ein weiteres besonders bevorzugtes Esteröl ist Triethylcitrat. Weitere erfindungsgemäß bevorzugte Produkte enthalten Triethylcitrat und mindestens ein $C_8$-$C_{16}$-Isoparaffin, ausgewählt aus Isononan, Isodecan, Isoundecan, Isododecan, Isotridecan, Isotetradecan, Isopentadecan und Isohexadecan sowie Mischungen dieser Isoparaffine. Weitere erfindungsgemäß bevorzugte Produkte enthalten Triethylcitrat und mindestens ein $C_8$-$C_{16}$-Isoparaffin, ausgewählt aus Isononan, Isodecan, Isoundecan, Isododecan, Isotridecan sowie Mischungen dieser $C_8$-$C_{16}$-Isoparaffine. Weitere erfindungsgemäß bevorzugte Produkte enthalten Triethylcitrat und eine Mischung aus Isodecan, Isoundecan, Isododecan und Isotridecan.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind 2-Hexyldecanol, 2-Octyldodecanol und 2-Ethylhexylalkohol. Ebenfalls bevorzugt ist Isostearylalkohol. Weitere bevorzugte nichtflüchtige Öle sind ausgewählt aus Mischungen aus Guerbetalkoholen und Guerbetalkoholestern, z. B. 2-Hexyldecanol und 2-Hexyldecyllaurat.

Der im Folgenden gebrauchte Ausdruck "Triglycerid" meint "Glycerintriester". Weitere erfindungsgemäß bevorzugte nichtflüchtige Öle sind ausgewählt aus den Triglyceriden von linearen oder verzweigten, gesättigten oder ungesättigten, gegebenenfalls hydroxylierten $C_{8-30}$-Fettsäuren, sofern diese unter Normalbedingungen flüssig sind. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Besonders bevorzugt sind synthetische Triglyceridöle, insbesondere Capric/Caprylic Triglycerides, z. B. die Handelsprodukte Myritol® 318 oder Myritol® 331 (BASF/ BASF) mit unverzweigten Fettsäureresten sowie Glyceryltrüsostearin und Glyceryltri(2-ethylhexanoat) mit verzweigten Fettsäureresten. Derartige Triglyceridöle machen bevorzugt einen Anteil von weniger als 50 Gew.-% am Gesamtgewicht aller kosmetischen Öle in der erfindungsgemäßen Zusammensetzung aus. Besonders bevorzugt beträgt das Gesamtgewicht an Triglyceridölen 0,1 - 3 Gew.-%, bevorzugt 0,2 - 1 Gew.-%, besonders bevorzugt 0,5 - 0,8 Gew.-%, jeweils bezogen auf die gesamte Zusammensetzung.

Weitere erfindungsgemäß besonders bevorzugte nichtflüchtige Nichtsiliconöle sind ausgewählt aus den Dicarbonsäureestern von linearen oder verzweigten $C_2$-$C_{10}$-Alkanolen, insbesondere Diisopropyladipat, Di-n-butyladipat,

Di-(2-ethylhexyl)adipat, Dioctyladipat, Diethyl-/Di-n-butyl/Dioctylsebacat, Düsopropylsebacat, Dioctylmalat, Dioctylmaleat, Dicaprylylmaleat, Düsooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den symmetrischen, unsymmetrischen oder cyclischen Estern der Kohlensäure mit $C_6$-$C_{20}$-Alkoholen, z. B. Di-n-caprylylcarbonat (Cetiol® CC) oder Di-(2-ethylhexyl)carbonat (Tegosoft DEC). Ester der Kohlensäure mit $C_1$-$C_5$-Alkoholen, z. B. Glycerincarbonat oder Propylencarbonat, sind hingegen keine als kosmetisches Öl geeigneten Verbindungen.

Weitere kosmetische Öle, die erfindungsgemäß besonders bevorzugt sind, sind ausgewählt aus nichtflüchtigen Siliconölen. Erfindungsgemäß bevorzugte nichtflüchtige Siliconöle sind ausgewählt aus linearen Polyalkylsiloxanen mit einer kinematischen Viskosität bei 25°C von mindestens 5 cSt bis 2000 cSt, insbesondere ausgewählt aus linearen Polydimethylsiloxanen mit einer kinematischen Viskosität bei 25°C von 5 cSt bis 2000 cSt, bevorzugt 10 bis 350 cSt, besonders bevorzugt 50 - 100 cSt, wie sie z. B. unter den Handelsnamen Dow Corning® 200 bzw. Xiameter PMX von Dow Corning bzw. Xiameter erhältlich sind. Weitere bevorzugte nichtflüchtige Siliconöle sind Phenyltrimethicone mit einer kinematischen Viskosität bei 25°C von 10 bis 100 cSt, bevorzugt von 15 - 30 cSt sowie Cetyldimethicone.

Erfindungsgemäß bevorzugte natürliche und synthetische Kohlenwasserstoffe sind ausgewählt aus Paraffinölen, Isohexadecan, Isoeicosan, Polyisobutenen und Polydecenen, die z. B. unter der Bezeichnung Emery® 3004, 3006, 3010 oder unter der Bezeichnung Nexbase® 2004G von Nestle erhältlich sind, sowie 1,3-Di-(2-ethylhexyl)-cyclohexan.

Als weitere obligatorische Komponente enthalten die erfindungsgemäßen Zusammensetzungen mindestens ein Hydrogel bildendes Polymer in einer Gesamtmenge von 0,08 - 1 Gew.-%, bevorzugt 0,1 - 0,8 Gew.-%, besonders bevorzugt 0,15 - 0,6 Gew.-%, außerordentlich bevorzugt 0,2 - 0,4 Gew.-%, jeweils bezogen auf das Gewicht der Zusammensetzung.

Erfindungsgemäß bevorzugte Hydrogel bildende Polymere sind ausgewählt aus Celluloseethern, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose, Methylhydroxyethylcellulose, weiterhin Xanthan-Gum, Sclerotium Gum, Succinoglucanen, Polygalactomannanen, insbesondere Guar-Gums und Johannisbrotkernmehl (Locust Bean Gum), insbesondere Guar-Gum und Locust Bean Gum selbst und den nichtionischen Hydroxyalkylguarderivaten und Johannisbrotkernmehl-Derivaten, wie Hydroxypropylguar, Carboxymethylhydroxypropylguar, Hydroxypropylmethylguar, Hydroxyethylguar und Carboxymethylguar, weiterhin Pectinen, Agar, Carragheen (Carrageenan), Traganth, Gummi arabicum, Karayagummi, Taragummi, Gellan, Gelatine, Casein, Propylenglycolalginat, Alginsäuren und deren Salze, insbesondere Natriumalginat, Kaliumalginat und Calciumalginat, weiterhin Polyvinylpyrrolidonen, Polyvinylalkoholen, Polyacrylamiden. Weiterhin kann der mindestens eine Hydrogelbildner - wenn auch weniger bevorzugt - ausgewählt sein aus physikalisch (z. B. durch Vorverkleisterung) und/oder chemisch modifizierten Stärken, insbesondere hydroxypropylierten Stärkephosphaten und Octenylstärkesuccinaten und deren Aluminium-, Calcium- oder Natriumsalzen, weiterhin - ebenfalls weniger bevorzugt - aus Acrylsäure-Acrylat-Copolymeren, Acrylsäure-Acrylamid-Copolymeren, Acrylsäure-Vinylpyrrolidon-Copolymeren, Acrylsäure-Vinylformamid-Copolymeren und Polyacrylaten.

Besonders bevorzugte Hydrogel bildende Polymere sind ausgewählt aus Celluloseethern, vor allem aus Hydroxyalkylcellulosen, insbesondere aus Hydroxypropylcellulose, Hydroxypropylmethylcellulose, Hydroxyethylcellulose, Carboxymethylcellulose, Cetylhydroxyethylcellulose, Hydroxybutylmethylcellulose und Methylhydroxyethylcellulose, sowie Mischungen hiervon. Ein außerordentlich bevorzugter Hydrogelbildner ist Hydroxyethylcellulose. Erfindungsgemäß außerordentlich bevorzugte Zusammensetzungen enthalten Hydroxyethylcellulose in einer Gesamtmenge von 0,08 - 1 Gew.-%, bevorzugt 0,1 - 0,8 Gew.-%, besonders bevorzugt 0,15 - 0,6 Gew.-%, außerordentlich bevorzugt 0,2 - 0,4 Gew.-%, jeweils bezogen auf das Gewicht der Zusammensetzung. Weitere erfindungsgemäß außerordentlich bevorzugte Zusammensetzungen enthalten als einzigen Hydrogelbildner Hydroxyethylcellulose in einer Gesamtmenge von 0,08 - 1 Gew.-%, bevorzugt 0,1 - 0,8 Gew.-%, besonders bevorzugt 0,15 - 0,6 Gew.-%, außerordentlich bevorzugt 0,2 - 0,4 Gew.-%, jeweils bezogen auf das Gewicht der Zusammensetzung.

Als weitere obligatorische Komponente enthalten die erfindungsgemäßen Zusammensetzungen in einer Gesamtmenge von 0,05 bis 4 Gew.-%, bevorzugt 0,5 - 3,5 Gew.-%, besonders bevorzugt 1 - 3 Gew.-% und außerordentlich bevorzugt 1,5 - 2,5 Gew.-%, mindestens einen Strukturanten, der ausgewählt ist aus linearen, gesättigten 1-Alkanolen mit 14 bis 22 C-Atomen, Glycerylmono- und -diestern von linearen oder verzweigten, gesättigten oder ungesättigten Carbonsäuren mit 8 bis 22 C-Atomen, linearen, gesättigten 1-Alkancarbonsäuren mit 14 bis 22 C-Atomen, Mono- und Diestern von Ethylenglycol mit linearen gesättigten und ungesättigten Fettsäuren mit 12 - 30, insbesondere 14 - 22 Kohlenstoffatomen, den Mono-, Di-, Tri- und Tetraestern von Pentaerythrit mit linearen gesättigten und ungesättigten Fettsäuren mit 12 - 30, insbesondere 14 - 22 Kohlenstoffatomen, sowie Mischungen dieser Strukturanten, wobei sich die Mengenangaben auf das Gewicht der Zusammensetzung beziehen.

Unter dem Begriff "Strukturant" wird im Sinne der vorliegenden Anmeldung ein nichtionischer Wasser-in-Öl-Emulgator mit einem HLB-Wert größer 1,0 und kleiner/gleich 7,0 verstanden, der in der Emulsion aufbaut, die die Viskosität erhöhen kann bzw. die zur Stabilisierung der Emulsion beiträgt. Dies kann über die Ausbildung von Wachspartikeln, eine Verfestigung der Ölphase oder durch die Ausbildung flüssigkristalliner, insbesondere lamellarer, Phasen

erfolgen.

Erfindungsgemäß besonders bevorzugte Strukturanten sind ausgewählt aus linearen, gesättigten 1-Alkanolen mit 14 bis 22 C-Atomen, insbesondere aus Myristylalkohol, Cetylalkohol, Stearylalkohol, Arachylalkohol und Behenylalkohol, sowie Mischungen dieser 1-Alkanole. Außerordentlich bevorzugt sind Stearylalkohol, Cetylalkohol, sowie Mischungen dieser 1-Alkanole, die auch als Cetearylalkohol bezeichnet werden. Als besonders vorteilhaft für die Temperaturstabilität bei 40°C der erfindungsgemäßen Emulsionen hat sich Stearylalkohol erwiesen, insbesondere Stearylalkohol in einer Menge von 1 - 3 Gew.-%, bezogen auf das Gewicht der Zusammensetzung.

Weitere erfindungsgemäß besonders bevorzugte Strukturanten sind ausgewählt aus Glycerylmono- und -diestern von linearen oder verzweigten, gesättigten oder ungesättigten Carbonsäuren mit 8 bis 22 C-Atomen, insbesondere aus Glycerylmonostearat, Glyceryldistearat, Glycerylmonocaprinat, Glyceryldicaprinat, Glycerylmonocaprylat, Glyceryldicaprylat, Glycerylmonolaurat, Glyceryldilaurat, Glycerylmonomyristat, Glyceryldimyristat, Glycerylmonopalmitat, Glyceryldipalmitat, Glycerylmono-12-hydroxystearat, Glyceryldi-12-hydroxystearat, Glycerylmonooleat, Glyceryldioleat, Glycerylmonolanolat, Glyceryldilanolat, Glycerylmonoisostearat und Glyceryldüsostearat, sowie Mischungen dieser Glycerylester. Aus dieser Strukturantenklasse außerordentlich bevorzugt sind Glycerylmonostearat, Glyceryldistearat, Glycerylmonopalmitat und Glyceryldipalmitat, sowie Mischungen dieser Glycerylester.

Als ebenfalls besonders vorteilhaft für die Temperaturstabilität bei 40°C der erfindungsgemäßen Emulsionen haben sich Glycerylmonostearat, Glyceryldistearat und Mischungen von Glycerylmonostearat und Glyceryldistearat erwiesen, insbesondere Mischungen von Glycerylmonostearat und Glyceryldistearat in einer Gesamtmenge von 1 - 3 Gew.-%, bezogen auf das Gewicht der Zusammensetzung.

Weitere erfindungsgemäß bevorzugte Strukturanten sind ausgewählt aus linearen, gesättigten 1-Alkancarbonsäuren mit 14 bis 22 C-Atomen, insbesondere aus Myristinsäure, Palmitinsäure, Stearinsäure, Arachinsäure und Behensäure, sowie Mischungen dieser 1-Alkancarbonsäuren. Weitere erfindungsgemäß bevorzugte Strukturanten sind ausgewählt aus den Mono- und Diestern von Ethylenglycol mit linearen gesättigten und ungesättigten Fettsäuren mit 12 - 30, insbesondere 14 - 22 Kohlenstoffatomen, insbesondere aus Ethylenglycolmonostearat, Ethylenglycoldistearat, Ethylenglycolmonocaprinat, Ethylenglycoldicaprinat, Ethylenglycolmonocaprylat, Ethylenglycoldicaprylat, Ethylenglycolmonolaurat, Ethylenglycoldilaurat, Ethylenglycolmonomyristat, Ethylenglycoldimyristat, Ethylenglycolmonopalmitat, Ethylenglycoldipalmitat, Ethylenglycolmono-12-hydroxystearat, Ethylenglycoldi-12-hydroxystearat, Ethylenglycolmonooleat, Ethylenglycoldioleat, Ethylenglycolmonolanolat und Ethylenglycoldilanolat, sowie Mischungen dieser Ethylenglycolester. Besonders bevorzugt sind Ethylenglycoldistearat, Ethylenglycolmonostearat, sowie Mischungen dieser beiden Ethylenglycolester.

Weitere erfindungsgemäß bevorzugte Strukturanten sind ausgewählt aus den Mono-, Di-, Tri- und Tetraestern von Pentaerythrit mit linearen gesättigten und ungesättigten Fettsäuren mit 12 - 30, insbesondere 14 - 22 Kohlenstoffatomen, insbesondere ausgewählt aus Pentaerythritylmonostearat, Pentaerythrityldistearat, Pentaerythritylmonocaprinat, Pentaerythrityldicaprinat, Pentaerythritylmonocaprylat, Pentaerythrityldicaprylat, Pentaerythritylmonolaurat, Pentaerythrityldilaurat, Pentaerythritylmonomyristat, Pentaerythrityldimyristat, Pentaerythritylmonopalmitat, Pentaerythrityldipalmitat, Pentaerythritylmono-12-hydroxystearat, Pentaerythrityldi-12-hydroxystearat, Pentaerythritylmonooleat, Pentaerythrityldioleat, Pentaerythritylmonolanolat und Pentaerythrityldilanolat, sowie Mischungen dieser Pentaerythritester. Besonders bevorzugt sind Pentaerythrityldistearat und Pentaerythritylmonostearat sowie Mischungen dieser Pentaerythritester, insbesondere Mischungen mit Pentaerythrityltristearat und/oder Pentaerythrityltetrastearat.

Als weitere obligatorische Komponente enthalten die erfindungsgemäßen Zusammensetzungen Wasser in einer Gesamtmenge von 30 - 90 Gew.-%, bevorzugt 40 - 80 Gew.-%, besonders bevorzugt 60 - 78 Gew.-%, außerordentlich bevorzugt 65 - 73 Gew.-%, jeweils bezogen auf das Gewicht der Zusammensetzung.

Der Gehalt an Kristallwasser, Hydratationswasser oder ähnlich molekular gebundenem Wasser, der in den eingesetzten Bestandteilen, insbesondere in den schweißhemmenden Wirkstoffen, enthalten ist, stellt im Sinne der vorliegenden Anmeldung kein freies Wasser dar und wird daher bei der Berechnung des vorstehend genannten Wassergehalts der Zusammensetzung nicht berücksichtigt.

In einer bevorzugten Ausführungsform der Erfindung ist ein Teil des Wassers durch ein wasserlösliches Lösemittel, wie insbesondere Ethanol, 1,2-Propylenglycol, Glycerin, 1,3-Butylenglycol und/oder ähnlichen Glycolen, sowie Mischungen dieser Lösemittel, ersetzt. Daher kann ein Wassergehalt von 45 - 58 Gew.-%, bevorzugt 50 - 55 Gew.-%, jeweils bezogen auf das Gewicht der Zusammensetzung, bevorzugt sein.

Die Zusammensetzung einiger bevorzugter erfindungsgemäßer Öl-in-Wasser-Emulsionen können den folgenden Tabellen entnommen werden. Die Mengenangaben beziehen sich, entsprechend dem Wortlaut von Anspruch 1, immer auf das Gesamtgewicht der jeweiligen Komponente in der Zusammensetzung (Gew.-%).

| | 1 | 2 | 3 | 4 |
|---|---|---|---|---|
| Schweißhemmendes Aluminiumsalz | 2 – 40 Gew.-% | 8 - 35 | 10 - 28 | 12 - 20 |
| Partialester von Tri-, Tetra- oder Penta-glycerin mit linearer gesättigter C8-22-Alkansäure und Genusssäure, | 0,1 – 2 | 0,3 – 1,5 | 0,5 – 1,1 | 0,6 – 0,8 |

| | | | | |
|---|---|---|---|---|
| HLB 9 - 15 | | | | |
| mind. ein N-Acyl-L-glutaminsäure-Natriumsalz der Formel (GLUT-1) | 0,1 – 2,0 | 0,3 – 1,2 | 0,6 – 1,1 | 0,8 – 1,0 |
| kosmet. Öl | 0,5 – 10 | 1 – 8 | 1,5 – 6 | 2 – 4,5 |
| mind. 1 Hydrogel bildendes Polymer | 0,08 – 1 | 0,1 – 0,8 | 0,15 – 0,6 | 0,2 – 0,4 |
| mind. 1 Strukturant gemäß Anspruch 1 | 0,05 bis 4 | 0,5 – 3,5 | 1 - 3 | 1,5 – 2,5 |
| Wasser | 30 - 90 | 40 - 80 | 60 - 78 | 65 – 73 |

| | 5 | 6 | 7 |
|---|---|---|---|
| Schweißhemmendes Aluminiumsalz | 2 – 40 Gew.-% | 8 - 35 | 10 - 28 |
| Partialester von Tri-, Tetra- oder Penta-glycerin mit linearer gesättigter C8-22-Alkansäure und Genusssäure, HLB 9 - 15 | 0,1 – 2 | 0,3 – 1,5 | 0,5 – 1,1 |
| mind. ein N-Acyl-L-glutaminsäure-Natriumsalz der Formel (GLUT-1) | 0,1 – 2,0 | 0,3 – 1,2 | 0,6 – 1,1 |
| kosmet. Öl | 0,5 – 10 | 1 – 8 | 2 – 4,5 |
| mind. 1 Hydrogel bildendes Polymer | 0,08 – 1 | 0,1 – 0,8 | 0,2 – 0,4 |
| mind. 1 Strukturant gemäß Anspruch 1 | 0,05 bis 4 | 0,5 – 3,5 | 1 - 3 |
| Wasser | 30 - 90 | 40 - 80 | 60 - 78 |

| | 8 | 9 | 10 | 11 |
|---|---|---|---|---|
| Schweißhemmendes Aluminiumsalz | 2 – 40 Gew.-% | 8 - 35 | 10 - 28 | 12 - 20 |
| Partialester von Tri-, | 0,1 – 2 | 0,3 – 1,5 | 0,5 – 1,1 | 0,6 – 0,8 |

| | | | | |
|---|---|---|---|---|
| Tetra- oder Penta-glycerin mit linearer gesättigter C8-22-Alkansäure und Genusssäure, HLB 9 - 15 | | | | |
| mind. ein N-Acyl-L-glutaminsäure-Natriumsalz der Formel (GLUT-1) | 0,1 – 2,0 | 0,3 – 1,2 | 0,6 – 1,1 | 0,8 – 1,0 |
| Gew.verhältnis von Gesamtgewicht an Polyglycerinester b) zu Gesamtgewicht an N-Acyl-L-glutaminsäure-Natriumsalz der Formel (GLUT-1) | 0,5 - 1,0 | 0,5 - 1,0 | 0,6 - 0,8 | 0,6 - 0,8 |
| kosmet. Öl | 0,5 – 10 | 1 – 8 | 1,5 – 6 | 2 – 4,5 |
| mind. 1 Hydrogel bildendes Polymer | 0,08 – 1 | 0,1 – 0,8 | 0,15 – 0,6 | 0,2 – 0,4 |
| mind. 1 Strukturant gemäß Anspruch 1 | 0,05 bis 4 | 0,5 – 3,5 | 1 - 3 | 1,5 – 2,5 |
| Wasser | 30 - 90 | 40 - 80 | 60 - 78 | 65 – 73 |

| | 12 | 13 | 14 | 15 |
|---|---|---|---|---|
| Schweißhemmendes Aluminiumsalz | 2 – 40 Gew.-% | 8 - 35 | 10 - 28 | 12 - 20 |
| Polyglyceryl-3 Dicitrate/ Stearate | 0,1 – 2 | 0,3 – 1,5 | 0,5 – 1,1 | 0,6 – 0,8 |
| N-Stearoylnatrium-L-glutamat | 0,1 – 2,0 | 0,3 – 1,2 | 0,6 – 1,1 | 0,8 – 1,0 |
| kosmet. Öl | 0,5 – 10 | 1 – 8 | 1,5 – 6 | 2 – 4,5 |
| Hydroxyethylcellulose | 0,08 – 1 | 0,1 – 0,8 | 0,15 – 0,6 | 0,2 – 0,4 |
| Stearylalkohol | 0,05 bis 4 | 0,5 – 3,5 | 1 - 3 | 1,5 – 2,5 |
| Wasser | 30 - 90 | 40 - 80 | 60 - 78 | 65 – 73 |

| | 16 | 17 | 18 | 19 |
|---|---|---|---|---|
| Schweißhemmendes Aluminiumsalz | 2 – 40 Gew.-% | 8 - 35 | 10 - 28 | 12 - 20 |
| Polyglyceryl-3 Dicitrate/Stearate | 0,1 – 2 | 0,3 – 1,5 | 0,5 – 1,1 | 0,6 – 0,8 |
| N-Stearoylnatrium-L-glutamat | 0,1 – 2,0 | 0,3 – 1,2 | 0,6 – 1,1 | 0,8 – 1,0 |
| Gew.verhältnis von Polyglyceryl-3 Dicitrate/Stearate zu N-Stearoylnatrium-L-glutamat | 0,5 - 1,0 | 0,5 - 1,0 | 0,6 - 0,8 | 0,6 - 0,8 |
| kosmet. Öl | 0,5 – 10 | 1 – 8 | 1,5 – 6 | 2 – 4,5 |
| Hydroxyethylcellulose | 0,08 – 1 | 0,1 – 0,8 | 0,15 – 0,6 | 0,2 – 0,4 |
| Stearylalkohol | 0,05 bis 4 | 0,5 – 3,5 | 1 - 3 | 1,5 – 2,5 |
| Wasser | 30 - 90 | 40 - 80 | 60 - 78 | 65 – 73 |

| | 20 | 21 | 22 |
|---|---|---|---|
| Schweißhemmendes Aluminiumsalz | 2 – 40 Gew.-% | 8 - 35 | 10 - 28 |
| Polyglyceryl-3 Dicitrate/ Stearate | 0,1 – 2 | 0,3 – 1,5 | 0,5 – 1,1 |
| N-Stearoylnatrium-L-glutamat | 0,1 – 2,0 | 0,3 – 1,2 | 0,6 – 1,1 |
| kosmet. Öl | 0,5 – 10 | 1 – 8 | 2 – 4,5 |
| Hydroxyethylcellulose | 0,08 – 1 | 0,1 – 0,8 | 0,2 – 0,4 |
| Stearylalkohol | 0,05 bis 4 | 0,5 – 3,5 | 1 - 3 |
| Wasser | 30 - 90 | 40 - 80 | 60 - 78 |

| | 23 | 24 | 25 |
|---|---|---|---|
| Schweißhemmendes Aluminiumsalz | 2 – 40 Gew.-% | 8 - 35 | 10 - 28 |
| Polyglyceryl-3 Dicitrate/ Stearate | 0,1 – 2 | 0,3 – 1,5 | 0,5 – 1,1 |
| N-Stearoylnatrium-L-glutamat | 0,1 – 2,0 | 0,3 – 1,2 | 0,6 – 1,1 |

| | | | |
|---|---|---|---|
| kosmet. Öl | 0,5 – 10 | 1 – 8 | 2 – 4,5 |
| Hydroxyethylcellulose | 0,08 – 1 | 0,1 – 0,8 | 0,2 – 0,4 |
| Stearylalkohol | 0,05 bis 4 | 0,5 – 3,5 | 1 - 3 |
| Wasser | 30 - 90 | 40 - 80 | 60 - 78 |
| Gew.verhältnis von Polyglyceryl-3 Dicitrate/Stearate zu N-Stearoylnatrium-L-glutamat | 0,5 - 1,0 | 0,5 - 1,0 | 0,6 - 0,8 |

Bevorzugte erfindungsgemäße Zusammensetzungen enthalten weiterhin mindestens ein wasserlösliches mehrwertiges $C_2$-$C_9$-Alkanol mit 2 - 6 Hydroxylgruppen sowie Mischungen hiervon. Ein derartiges Polyol verbessert nicht nur die kosmetischen Pflegeeigenschaften der erfindungsgemäßen Zusammensetzungen, sondern wirkt sich auch fördernd auf die Temperaturstabilität der Emulsion bei 40 °C aus. Bevorzugt sind die Polyole ausgewählt aus 1,2-Propylenglycol, Glycerin, 2-Methyl-1,3-propandiol, 1,2-Butylenglycol, 1,3-Butylenglycol, 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebigen Isomeren-Gemischen von cis- und trans-1,4-Dimethylolcyclohexan, sowie Mischungen der vorgenannten Substanzen. Besonders bevorzugte erfindungsgemäße Zusammensetzungen enthalten mindestens ein wasserlösliches mehrwertiges $C_2$-$C_9$-Alkanol mit 2 - 6 Hydroxylgruppen in einer Gesamtmenge von 0,5 - 14 Gew.-%, bevorzugt 2 - 11 Gew.-%, besonders bevorzugt 5 - 7 Gew.-%, enthalten ist, jeweils bezogen auf das Gesamtgewicht der Zusammensetzung.

Besonders bevorzugte erfindungsgemäße Zusammensetzungen enthalten weiterhin mindestens einen Riechstoff. Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems.

Als Riechstoffe können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und die Terpineole alpha-Terpineol, beta-Terpineol, gamma-Terpineol und delta-Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Die Riechstoffe können auch in verkapselter Form enthalten sein. Besonders bevorzugte erfindungsgemäße Zusammensetzungen enthalten mindestens einen Riechstoff in einer Gesamtmenge von 0,00001 bis 10 Gew.-%, bevorzugt 0,5 - 7 Gew.-%, außerordentlich bevorzugt 1 - 6 Gew.-%, jeweils bezogen auf das Gesamtgewicht der Zusammensetzung.

Weitere erfindungsgemäß bevorzugte Zusammensetzungen enthalten mindestens einen Deodorant-Wirkstoff in einer Gesamtmenge von 0,001 - 10 Gew.-%, bevorzugt 0,2 - 7 Gew.-%, besonders bevorzugt 1 - 5 Gew.-%, außerordentlich bevorzugt 1,5 - 3 Gew.-%, wobei sich die Gew.-%-Angaben auf das Gesamtgewicht der Zusammensetzung beziehen.

Ethanol gilt erfindungsgemäß nicht als Deodorant-Wirkstoff, sondern, sofern vorhanden, nur als Bestandteil des Trägers.

In einer bevorzugten Ausführungsform enthalten die erfindungsgemäßen Zusammensetzungen als deodorierenden Wirkstoff mindestens ein Silbersalz, das bevorzugt ausgewählt ist aus Silbersulfat, Silbernitrat, Silbercitrat, Silberdihydrogencitrat, Silberlactat, Silberacetat, Silbermalat, Silbersuccinat, Silbertartrat, Silbermandelat, Silbersalicylat, Silbergluconat, Silberadipat und Silbergalactarat, sowie aus Mischungen dieser Salze. Außerordentlich bevorzugt sind Silbersulfat, Silbercitrat, Silberdihydrogencitrat und Silberlactat, sowie Mischungen dieser Salze.

Weitere bevorzugte erfindungsgemäße Zusammensetzungen enthalten mindestens ein Silbersalz, das bevorzugt ausgewählt ist aus Silbersulfat, Silbernitrat, Silbercitrat, Silberdihydrogencitrat, Silberlactat, Silberacetat, Silbermalat, Silbersuccinat, Silbertartrat, Silbermandelat, Silbersalicylat, Silbergluconat, Silberadipat und Silbergalactarat, sowie aus Mischungen dieser Salze, in solchen Mengen, dass Silber in einer Gesamtmenge von 1 - 100 ppm, bevorzugt 2 - 50 ppm, besonders bevorzugt 5 - 20 ppm, außerordentlich bevorzugt 7 - 10 ppm, jeweils bezogen auf das Gewicht der treibmittelfreien Zusammensetzung, enthalten ist. Anhand der Molmassen von Silber (107,87 g/mol) und den jeweiligen Silbersalzen - Silberlactat z. B. hat eine Molmasse von 196,94 g/mol - kann die entsprechend nötige Menge an Silbersalz(en) berechnet werden.

In einer weiteren bevorzugten Ausführungsform enthalten die erfindungsgemäßen Zusammensetzungen als deodorierenden Wirkstoff mindestens einen aromatischen Alkohol der Struktur (AA-1),

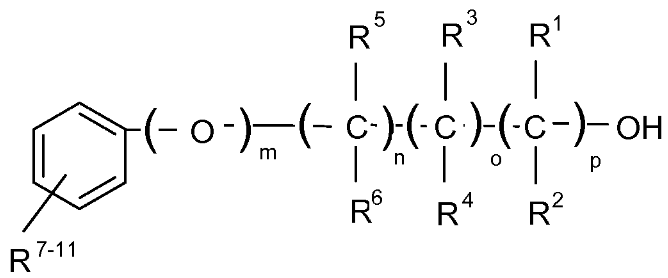

(AA-1)

wobei

die Reste $R^1$ bis $R^6$ unabhängig voneinander ein Wasserstoffatom, eine Alkylgruppe mit 1 bis 10 Kohlenstoffatomen, die linear oder verzweigt und substituiert sein können mit OH-Gruppen oder Alkoxy-Gruppen mit 1 bis 5 Kohlenstoffatomen, oder eine Alkenylgruppe mit 2 bis 10 Kohlenstoffatomen, die linear oder verzweigt und substituiert sein können mit OH-Gruppen oder Alkoxy-Gruppen mit 1 bis 5 Kohlenstoffatomen,

die Reste $R^7$ bis $R^{11}$ unabhängig voneinander ein Wasserstoffatom, ein Halogenatom, insbesondere ein Chloratom, oder eine Alkylgruppe mit 1 bis 10 Kohlenstoffatomen, die linear oder verzweigt und substituiert sein können mit OH-Gruppen oder Alkoxy-Gruppen mit 1 bis 5 Kohlenstoffatomen, insbesondere mit einer Methoyxgruppe,

m = 0 oder 1 ist, n, o, p = unabhängig voneinander ganze Zahlen von 0 bis 10 sind, wobei mindestens einer der Werte n, o, p $\neq$ 0 ist.

Besonders bevorzugte erfindungsgemäße Produkte enthalten mindestens einen Alkohol AA-1, wie vorstehend beschrieben, der ausgewählt ist aus Anisalkohol, 2-Methyl-5-phenyl-pentan-1-ol, 1,1-Dimethyl-3-phenyl-propan-1-ol, Benzylalkohol, 2-Phenylethan-1-ol, 3-Phenylpropan-1-ol, 4-Phenylbutan-1-ol, 5-Phenylpentan-1-ol, 2-Benzylheptan-1-ol, 2,2-Dimethyl-3-phenylpropan-1-ol, 2,2-Dimethyl-3-(3'-methylphenyl)-propan-1-ol, 2-Ethyl-3-phenylpropan-1-ol, 2-Ethyl-3-(3'-methylphenyl)-propan-1-ol, 3-(3'-Chlorphenyl)-2-ethylpropan-1-ol, 3-(2'-Chlorphenyl)-2-ethylpropan-1-ol, 3-(4'-Chlorphenyl)-2-ethylpropan-1-ol, 3-(3',4'-Dichlorphenyl)-2-ethylpropan-1-ol, 2-Ethyl-3-(2'-methylphenyl)-propan-1-ol, 2-Ethyl-3-(4'-methylphenyl)-propan-1-ol, 3-(3',4'-Dimethylphenyl)-2-ethylpropan-1-ol, 2-Ethyl-3-(4'-methoxyphenyl)-propan-1-ol, 3-(3',4'-Dimethoxyphenyl)-2-ethyl-propan-1-ol, 2-Allyl-3-phenylpropan-1-ol und 2-n-Pentyl-3-phenylpropan-1-ol sowie Mischungen hiervon. Außerordentlich bevorzugt ist 2-Benzylheptan-1-ol sowie Mischungen von 2-Benzylheptan-1-ol und Phenoxyethanol. Weitere besonders bevorzugte erfindungsgemäße Zusammensetzungen enthalten mindestens einen Alkohol AA-1, wie vorstehend beschrieben, in einer Gesamtmenge von 0,05 - 10 Gew.-%, bevorzugt 0,1 - 5 Gew.-%, besonders bevorzugt 0,2 - 2 Gew.-%, außerordentlich bevorzugt 0,3 - 1,5 Gew.-%, jeweils bezogen auf das Gewicht der Zusammensetzung. Außerordentlich bevorzugte erfindungsgemäße Mittel enthalten 2-Benzylheptan-1-ol in einer Gesamtmenge von 0,05 - 1,5 Gew.-%, bevorzugt 0,1 - 1 Gew.-%, besonders bevorzugt 0,2 - 0,5 Gew.-%, jeweils bezogen auf das Gewicht der Zusammensetzung. Auch die Alkohole AA-1 können in verkapselter Form enthalten sein.

In einer weiteren bevorzugten Ausführungsform enthalten die erfindungsgemäßen Zusammensetzungen als

Deodorant-Wirkstoff mindestens ein 1,2-Alkandiol mit 5 bis 12 C-Atomen, welches durch die Formel HO-$CH_2$-CH(OH)-$(CH_2)_n$-$CH_3$ beschrieben wird, in der n für die Zahlen 2, 3, 4, 5, 6, 7, 8 oder 9 steht, sowie Mischungen dieser 1,2-Alkandiole. Erfindungsgemäß besonders bevorzugte 1,2-Alkandiole mit 5 bis 12 C-Atomen sind ausgewählt aus 1,2-Pentandiol, 1,2-Hexandiol, 1,2-Octandiol, 1,2-Decandiol und Mischungen hiervon. Eine ganz besonders bevorzugte erfindungsgemäße Kombination sind Mischungen aus 1,2-Hexandiol und 1,2-Octandiol, vorzugsweise im Gewichtsverhältnis 10:1 bis 1:10, weiter bevorzugt von 5:1 bis 1:5, besonders bevorzugt im Gewichtsverhältnis 1:1. Bevorzugte erfindungsgemäße Zusammensetzungen enthalten mindestens ein 1,2-Alkandiol mit 5 bis 12 C-Atomen, das durch die Formel HO-$CH_2$-CH(OH)-$(CH_2)_n$-$CH_3$ beschrieben wird, in der n für die Zahlen 2, 3, 4, 5, 6, 7, 8 oder 9 steht, in einer Gesamtmenge von 0,2 - 15 Gew.-%, bevorzugt 0,3 - 10 Gew.-%, besonders bevorzugt 0,4 - 5 Gew.-% und außerordentlich bevorzugt 0,5 - 2 Gew.-%, jeweils bezogen auf das Gewicht der Zusammensetzung. Außerordentlich bevorzugte erfindungsgemäße Zusammensetzungen enthalten 0,2 - 0,5 Gew.-% 1,2-Hexandiol und 0,2 - 0,5 Gew.-% 1,2-Octandiol, jeweils bezogen auf das Gewicht der Zusammensetzung.

Weitere bevorzugte erfindungsgemäße Mittel sind gekennzeichnet durch einen Gehalt an dem Deodorant-Wirkstoff 3-(2-Ethylhexyloxy)-1,2-propandiol, bevorzugt in einer Gesamtmenge von 0,05 - 5 Gew.-%, bevorzugt 0,1 - 2 Gew.-%, besonders bevorzugt 0,2 - 1,5 Gew.-%, außerordentlich bevorzugt 0,5 - 1,0 Gew.-%, jeweils bezogen auf das Gewicht der Zusammensetzung. Weitere bevorzugte erfindungsgemäße Mittel sind gekennzeichnet durch einen Gehalt an Tropolon (2-Hydroxy-2,4,6-Cycloheptatrienon), bevorzugt in einer Menge von 0,001 - 0,1 Gew.-%, bezogen auf das Gewicht der Zusammensetzung.

Weitere bevorzugte erfindungsgemäße Mittel sind gekennzeichnet durch einen Gehalt an dem Deodorant-Wirkstoff Triethylcitrat. Triethylcitrat ist ein bekannter Deodorantwirkstoff, der als Enzyminhibitor für Esterasen und Lipasen wirkt und somit zur Breitbandwirkung erfindungsgemäßer Mittel beiträgt. Bevorzugte erfindungsgemäße Mittel enthalten 0,5 - 15 Gew.-%, bevorzugt 3 - 8 Gew.-%, außerordentlich bevorzugt 4 - 6 Gew.-%, jeweils bezogen auf das Gewicht der Zusammensetzung.

Verbindungen, die mindestens eines der Enzyme Arylsulfatase, beta-Glucuronidase, 5-Lipoxigenase oder Cystathionin-beta-Lyase inhibieren, stellen ebenfalls erfindungsgemäß bevorzugte Deodorantwirkstoffe dar.

Weitere bevorzugte erfindungsgemäße Zusammensetzungen sind gekennzeichnet durch einen Gehalt an mindestens einer Verbindung, die ein Inhibitor des Enzyms Arylsulfatase ist. Erfindungsgemäß bevorzugte Deodorantwirkstoffe, die als Arylsulfatase-Inhibitor wirken, sind solche, wie sie in US 5643559, US 5676937, WO2001/099376 A2, EP 1430879 A1 und DE 10216368 A1 offenbart sind. Weitere besonders bevorzugte erfindungsgemäße Mittel sind gekennzeichnet durch einen Gehalt an mindestens einer Verbindung, die ein Inhibitor des Enzyms Arylsulfatase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Zusammensetzung.

Weitere bevorzugte erfindungsgemäße Mittel sind gekennzeichnet durch einen Gehalt an mindestens einer Verbindung, die ein Inhibitor des Enzyms beta-Glucuronidase ist. Erfindungsgemäß bevorzugte Deodorantwirkstoffe, die als beta-Glucuronidase-Inhibitor wirken, sind solche, wie sie in WO2003/039505 A2 offenbart sind. Weitere besonders bevorzugte erfindungsgemäße Mittel sind gekennzeichnet durch einen Gehalt an mindestens einer Verbindung, die ein Inhibitor des Enzyms beta-Glucuronidase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Zusammensetzung.

Weitere bevorzugte erfindungsgemäße Mittel sind gekennzeichnet durch einen Gehalt an mindestens einer Verbindung, die ein Inhibitor des Enzyms Lipase ist. Erfindungsgemäß bevorzugte Deodorantwirkstoffe, die als Lipase-Inhibitor wirken, sind ausgewählt aus denen, die in EP 1428520 A2 offenbart sind, weiterhin ausgewählt aus den Aminomethylenmalonsäure-Derivaten gemäß DE 3018132 A1, den Ethylenoxid-Propylenoxid-Copolymeren gemäß GB 2335596 A1 und den Salzen der Phytinsäure gemäß EP 650 720 A1. Weitere besonders bevorzugte erfindungsgemäße Mittel sind gekennzeichnet durch einen Gehalt an mindestens einer Verbindung, die ein Inhibitor des Enzyms Lipase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Zusammensetzung.

Weitere besonders bevorzugte erfindungsgemäße Mittel sind gekennzeichnet durch einen Gehalt an mindestens einer Verbindung, die ein Inhibitor der 5-Lipoxigenase ist. Erfindungsgemäß bevorzugte Deodorantwirkstoffe, die als 5-Lipoxigenase-Inhibitor wirken, sind in EP 1428519 A2 offenbart. Weitere besonders bevorzugte erfindungsgemäße Mittel sind gekennzeichnet durch einen Gehalt an mindestens einer Verbindung, die ein Inhibitor des Enzyms 5-Lipoxigenase ist, in einer Gesamtmenge von 0,001 - 10 Gew.-%, bevorzugt 0,01 - 5 Gew.-%, besonders bevorzugt 0,1 - 2,5 Gew.-%, jeweils bezogen auf das Gewicht der Zusammensetzung.

Weitere besonders bevorzugte erfindungsgemäße Mittel sind gekennzeichnet durch einen Gehalt an mindestens einer Verbindung, die ein Inhibitor des Enzyms Cystathionin-beta-Lyase ist. Erfindungsgemäß bevorzugte Deodorantwirkstoffe, die als Inhibitor der Cystathionin-beta-Lyase wirken, sind ausgewählt aus denen, die in EP 495918 B1, WO 2006/079934, DE 102010000746 A1, WO2010/031657 A1 und WO2010/046291 A1 offenbart sind. Weitere besonders bevorzugte erfindungsgemäße Mittel sind gekennzeichnet durch einen Gehalt an mindestens einer Verbindung, die ein Inhibitor des Enzyms Cystathionin-beta-Lyase ist, in einer Gesamtmenge von 0,001 - 10 Gew.-%, bevorzugt

0,01 - 5 Gew.-%, besonders bevorzugt 0,1 - 2,5 Gew.-%, jeweils bezogen auf das Gewicht der Zusammensetzung.

Weitere besonders bevorzugte erfindungsgemäße Zusammensetzungen sind gekennzeichnet durch einen Gehalt an mindestens einem kationischen Phospholipid der Formel KPL,

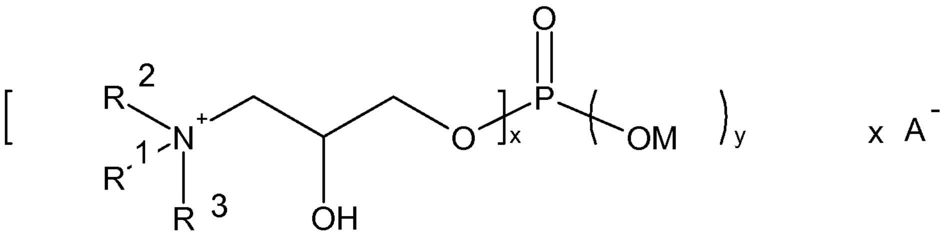

KPL

in der $R^1$ eine Alkyl-, Alkenyl- oder Hydroxyalkylgruppe mit 8 bis 22 C-Atomen oder eine Acylaminoalkylgruppe der Formel $R^5CONH(C_mH_{2m})$- ist, worin $R^5CO$ eine lineare Acylgruppe mit 8 bis 22 C-Atomen und m = 2 oder 3 ist, $R^2$ und $R^3$ Alkylgruppen mit 1 bis 4 C-Atomen oder Hydroxyalkylgruppen mit 2 bis 4 C- Atomen oder Carboxyalkylgruppen der Formel $-(CH_2)_z$-COOM sind, worin z einen Wert von 1 bis 3 hat und M Wasserstoff oder ein Alkalimetallkation ist, x einen Wert von 1 bis 3 und y einen Wert von (3 - x) hat, M Wasserstoff oder ein Alkalimetallkation ist und $A^-$ ein Anion ist.

Bevorzugte Alkylgruppen mit 8 bis 22 C-Atomen sind ausgewählt aus einer n-Octyl-, n-Nonyl-, n-Decyl-, n-Undecyl-, Lauryl-, n-Tridecanyl-, Myristyl-, n-Pentadecanyl-, Cetyl-, Palmityl-, Stearyl-, Elaidyl-, Arachidyl-, Behenyl- und einer Cocyl-Gruppe. Eine repräsentative Cocyl-Gruppe besteht, bezogen auf ihr Gesamtgewicht, aus 4 - 9 Gew.-% n-Octyl-, 4 - 9 Gew.-% n-Decyl-, 45 - 55 Gew.-% Lauryl-, 15 - 21 Gew.-% Myristyl-, 8 - 13 Gew.-% Palmityl- und 7 - 14 Gew.-% Stearyl-Gruppen. Bevorzugte Alkenylgruppen mit 8 bis 22 C-Atomen sind ausgewählt aus einer Linoleyl-Gruppe ((9Z,12Z)-Octadeca-9,12-dien-1-yl) und einer Linolenyl-Gruppe ((9Z,12Z,15Z)-Octadeca-9,12,15-trien-1-yl). Eine bevorzugte Hydroxyalkylgruppe mit 8 bis 22 C-Atomen ist ausgewählt aus einer 12-Hydroxystearylgruppe.

Besonders bevorzugte kationische Phospholipide der Formel KPL sind solche, bei denen $R^1$ eine Acylaminoalkylgruppe der Formel $R^5CONH(C_mH_{2m})$- ist, worin $R^5CO$ eine lineare Acylgruppe mit 8 bis 22 C-Atomen darstellt und m = 3 ist.

Bevorzugte erfindungsgemäße Zusammensetzungen enthalten als deodorierenden Wirkstoff ein kationisches Phospholipid der Formel KPL,

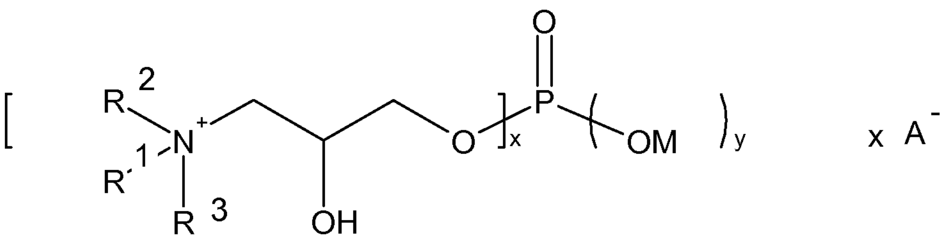

KPL

in der $R^1$ eine Acylaminoalkylgruppe der Formel $R^5CONH(C_mH_{2m})$- ist, worin $R^5CO$ ausgewählt ist aus einer Cocoylgruppe, einer Lauroylgruppe, einer Myristoylgruppe und einer Linoleoyl-Gruppe und m = 3 ist, $R^2$ und $R^3$ Methylgruppen sind, x = 2, y = 1, M ein Natriumion und $A^-$ ein Chloridion sind. Bevorzugt ist mindestens ein kationisches Phospholipid der Formel KPL mit den vorstehend genannten Merkmalen in einer Gesamtmenge von 0,05 - 2 Gew.-%, bevorzugt 0,1 - 1 Gew.-%, besonders bevorzugt 0,15 - 0,4 Gew.-%, enthalten, jeweils bezogen auf das Gewicht der Zusammensetzung.

Besonders bevorzugte erfindungsgemäße Zusammensetzungen enthalten ein kationisches Phospholipid der Formel KPL,

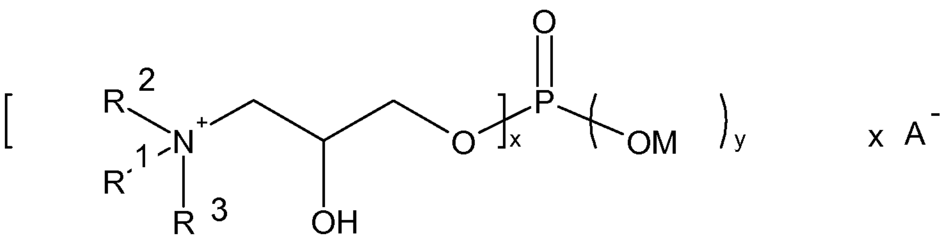

KPL

in der $R^1$ eine Cocoylaminopropylgruppe (auch als Cocamidopropylgruppe bezeichnet) ist, $R^2$ und $R^3$ Methylgruppen sind, x = 2, y = 1, M ein Natriumion und $A^-$ ein Chloridion sind und die unter der INCI-Bezeichnung Cocamidopropyl PG-Dimonium Chloride Phosphate erhältlich ist, in einer Gesamtmenge von 0,05 - 2 Gew.-%, bevorzugt 0,1 - 1 Gew.-%, besonders bevorzugt 0,15 - 0,4 Gew.-%, jeweils bezogen auf das Gewicht der Zusammensetzung.

Weitere besonders bevorzugte erfindungsgemäße Zusammensetzungen enthalten ein kationisches Phospholipid der Formel KPL,

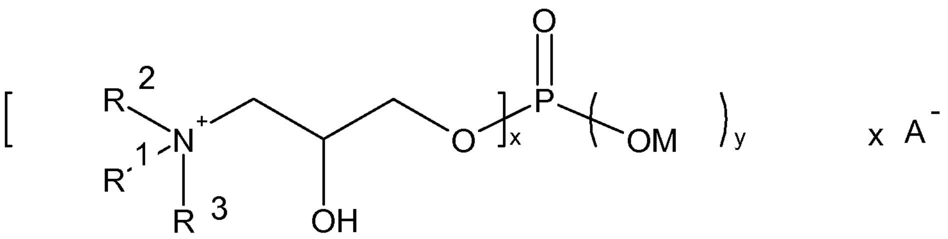

KPL

in der $R^1$ eine Myristoylaminopropylgruppe ist, $R^2$ und $R^3$ Methylgruppen sind, x = 2, y = 1, M ein Natriumion und $A^-$ ein Chloridion sind und die unter der INCI-Bezeichnung Myristamidopropyl PG-Dimonium Chloride Phosphate erhältlich ist, in einer Gesamtmenge von 0,05 - 2 Gew.-%, bevorzugt 0,1 - 1 Gew.-%, besonders bevorzugt 0,15 - 0,4 Gew.-%, jeweils bezogen auf das Gewicht der Zusammensetzung.

Weitere besonders bevorzugte erfindungsgemäße Zusammensetzungen enthalten ein kationisches Phospholipid der Formel KPL,

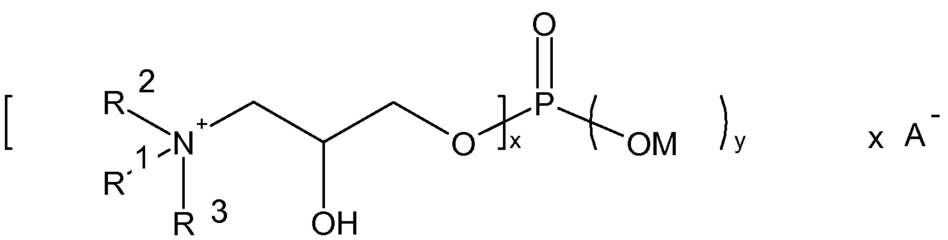

KPL

in der $R^1$ eine Lauroylaminopropylgruppe ist, $R^2$ und $R^3$ Methylgruppen sind, x = 2, y = 1, M ein Natriumion und $A^-$ ein Chloridion sind, in einer Gesamtmenge von 0,05 - 2 Gew.-%, bevorzugt 0,1 - 1 Gew.-%, besonders bevorzugt 0,15 - 0,4 Gew.-%, jeweils bezogen auf das Gewicht der Zusammensetzung.

Weitere erfindungsgemäß bevorzugte Deodorant-Wirkstoffe sind Geruchsabsorber, desodorierend wirkende Ionenaustauscher, keimhemmende Mittel, präbiotisch wirksame Komponenten sowie Enzyminhibitoren oder, besonders bevorzugt, Kombinationen der genannten Wirkstoffe.

Silicate dienen als Geruchsabsorber, die auch gleichzeitig die rheologischen Eigenschaften der erfindungsgemäßen Zusammensetzung vorteilhaft unterstützen. Zu den erfindungsgemäß besonders bevorzugten Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere bevorzugte Geruchsabsorber sind beispielsweise Zeolithe, Zinkricinoleat, Cyclodextrine, bestimmte Metalloxide, wie z. B. Aluminiumoxid, sowie Chlorophyll. Sie sind bevorzugt in einer Gesamtmenge von 0,1 - 10 Gew.-%, besonders bevorzugt 0,5 - 7 Gew.-% und außerordentlich bevorzugt 1 - 5 Gew.-%, jeweils bezogen auf die Gesamtzusammensetzung, enthalten.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzethoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, sowie Carbonsäureester des Mono-, Di- und Triglycerins (z. B. Glycerinmonolaurat, Diglycerinmonocaprinat) bevorzugte Deodorant-Wirkstoffe.

Bevorzugte erfindungsgemäße Zusammensetzungen enthalten mindestens einen Deodorant-Wirkstoff, der ausgewählt ist aus Silbersalzen, aromatischen Alkoholen der Struktur AA-1 mit den vorstehend genannten Substituenten, 1,2-Alkandiolen mit 5 bis 12 Kohlenstoffatomen, alpha-(2-Ethylhexyl)glycerinether (3-(2-Ethylhexlyoxy)-1,2-propandiol), Tropolon, Triethylcitrat, kationischen Phospholipiden der Formel KPL mit den vorstehend genannten Substituenten, sowie Mischungen hiervon.

Weitere erfindungsgemäße Zusammensetzungen können zur Verbesserung der Hautverträglichkeit mindestens ein Vitamin oder ein Vitaminderivat enthalten. Bevorzugt sind hier Panthenol, Niacinamid, L-Ascorbinsäure, L-Ascorbinsäureester, wie Ascorbylpalmitat, oder Magnesiumascorbylphosphat oder Natriumascorbylphosphat, 3-O-Ethylascorbinsäure, 3-O-Cetylascorbinsäure, 2-O-Ethylascorbinsäure, Retinylpalmitat, Tocopherol, Tocopherylacetat, sowie Mischungen dieser Substanzen. Bevorzugt ist mindestens ein Vitamin oder ein Vitaminderivat in einer Gesamtmenge von 0,01 - 2 Gew.-%, bevorzugt 0,1 - 1,5 Gew.-%, besonders bevorzugt 0,5 - 1 Gew.-%, jeweils bezogen auf das Gewicht der Zusammensetzung, enthalten.

Antioxidantien zur Verbesserung der Lagerstabilität wie auch als pflegende Inhaltsstoffe können in bevorzugten erfindungsgemäßen Zusammensetzungen ebenfalls enthalten sein. Bevorzugte Antioxidantien sind Tocopherol, Tocopherylacetat, L-Ascorbinsäureester, wie Ascorbylpalmitat, oder Magnesiumascorbylphosphat oder Natriumascorbylphosphat, 3-O-Ethylascorbinsäure, 3-O-Cetylascorbinsäure, 2-O-Ethylascorbinsäure, Lipochroman-6, sowie Mischungen dieser Substanzen. Bevorzugt ist mindestens ein Antioxidans in einer Gesamtmenge von 0,001 - 1 Gew.-%, bevorzugt 0,01 - 0,6 Gew.-%, besonders bevorzugt 0,1 - 0,3 Gew.-%, jeweils bezogen auf das Gewicht der Zusammensetzung, enthalten.

Als Schutz vor Textilverfärbungen, die durch die erfindungsgemäßen Zusammensetzungen verursacht werden könnten, enthalten bevorzugte erfindungsgemäße Zusammensetzungen mindestens eine Pyridindicarbonsäure, bevorzugt 2,6-Pyridindicarbonsäure, besonders bevorzugt in einer Menge von 0,01 - 1 Gew.-%, bevorzugt 0,02 - 0,8 Gew.-%, besonders bevorzugt 0,05 - 0,6 Gew.-%, noch weiter bevorzugt 0,1 - 0,4 Gew.-% und insbesondere 0,2 - 0,3 Gew.-%, wobei sich die Gew.-%-Angaben jeweils auf das Gesamtgewicht der Zusammensetzung beziehen

Um das Erscheinungsbild rasierter Achselhaut nachhaltig zu verbessern, enthalten bevorzugte erfindungsgemäße Zusammensetzungen mindestens eine Haarwuchs inhibierende Substanz und oder eine das Haar aufhellende Substanz, besonders bevorzugt jeweils in einer Gesamtmenge von 0,0001 - 1 Gew.-%, weiter bevorzugt 0,001 - 0,6 Gew.-%, außerordentlich bevorzugt 0,02 - 0,2 Gew.-%, jeweils bezogen auf das Gewicht der Zusammensetzung.

Als Pflegestoffe zur Verbesserung der Hautverträglichkeit enthalten weitere bevorzugte erfindungsgemäße Zusammensetzungen mindestens ein Protein, eine Aminosäure oder ein Peptid, beispielsweise Yoghurtprotein, besonders bevorzugt in einer Gesamtmenge von 0,0001 - 1 Gew.-%, weiter bevorzugt 0,001 - 0,6 Gew.-%, außerordentlich bevorzugt 0,02 - 0,2 Gew.-%, bezogen auf das Gewicht der Zusammensetzung.

Weitere bevorzugte erfindungsgemäße Zusammensetzungen enthalten mindestens einen festen anorganischen Emulsionsstabilisator, ausgewählt aus Silica, hydrophobem Silica, Mica (Mica auch zur Erzielung eines optischen Perlglanzeffekts) sowie Mischungen dieser anorganischen Substanzen, bevorzugt in einer Gesamtmenge von 0,01 - 1 Gew.-%, weiter bevorzugt 0,05 - 0,6 Gew.-%, außerordentlich bevorzugt 0,1 - 0,4 Gew.-%, bezogen auf das Gewicht der Zusammensetzung.

Weitere bevorzugte erfindungsgemäße Zusammensetzungen enthalten mindestens ein Konservierungsmittel.

Ein weiterer Gegenstand der vorliegenden Anmeldung ist ein Verfahren zur nicht-therapeutischen, kosmetischen schweißhemmenden Behandlung des Körpers, bei dem eine schweißhemmende Zusammensetzung in Form einer Öl-in-Wasser-Emulsion, die keine Mikroemulsion ist, enthaltend

a) mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in dem Mittel

beziehen, und zusätzlich dazu
b) in einer Gesamtmenge von 0,1 - 2 Gew.-%, bevorzugt 0,3 - 1,5 Gew.-%, besonders bevorzugt 0,5 - 1,1 Gew.-%, außerordentlich bevorzugt 0,6 - 0,8 Gew.-%, mindestens eine oberflächenaktive Verbindung mit einem HLB-Wert im Bereich von 9 bis 15, ausgewählt aus den Partialestern eines Polyglycerins, das 3, 4 oder 5 Glycerin-Einheiten umfasst, mit einer linearen oder verzweigten, gesättigten oder ungesättigten Carbonsäure mit 8 bis 22 C-Atomen und mit einer organischen Genusssäure, und zusätzlich dazu
c) in einer Gesamtmenge von 0,1 - 2,0 Gew.-%, bevorzugt 0,3 - 1,2 Gew.-%, besonders bevorzugt 0,6 - 1,1 Gew.-%, außerordentlich bevorzugt 0,8 - 1,0 Gew.-%, mindestens ein N-Acyl-L-glutaminsäure-Natriumsalz der Formel (GLUT-1)

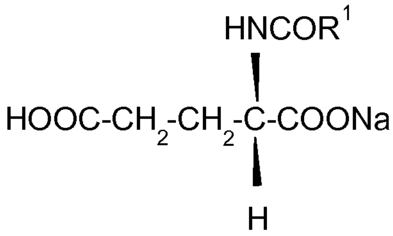

(GLUT-1),

worin $R^1CO$ eine lineare oder verzweigte, gesättigte oder ungesättigte Acylgruppe mit 6 bis 22 C-Atomen, bevorzugt mit 8 bis 18 C-Atomen, darstellt;
und zusätzlich dazu
d) mindestens ein unter Normalbedingungen flüssiges kosmetisches Öl, das kein Riechstoff und kein ätherisches Öl ist, in einer Gesamtmenge von 0,5 - 10 Gew.-%, bevorzugt 1 - 8 Gew.-%, besonders bevorzugt 1,5 - 6 Gew.-%, außerordentlich bevorzugt 2 - 4,5 Gew.-%,
und zusätzlich dazu
e) mindestens ein Hydrogel bildendes Polymer in einer Gesamtmenge von 0,08 - 1 Gew.-%, bevorzugt 0,1 - 0,8 Gew.-%, besonders bevorzugt 0,15 - 0,6 Gew.-%, außerordentlich bevorzugt 0,2 - 0,4 Gew.-%,
und zusätzlich dazu
f) in einer Gesamtmenge von 0,05 bis 4 Gew.-%, bevorzugt 0,5 - 3,5 Gew.-%, besonders bevorzugt 1 - 3 Gew.-% und außerordentlich bevorzugt 1,5 - 2,5 Gew.-%, mindestens einen Strukturanten, ausgewählt aus linearen, gesättigten 1-Alkanolen mit 14 bis 22 C-Atomen, Glycerylmono- und -diestern von linearen oder verzweigten, gesättigten oder ungesättigten Carbonsäuren mit 8 bis 22 C-Atomen, linearen, gesättigten 1-Alkancarbonsäuren mit 14 bis 22 C-Atomen, Mono- und Diestern von Ethylenglycol mit linearen gesättigten und ungesättigten Fettsäuren mit 12 - 30, insbesondere 14 - 22 Kohlenstoffatomen, den Mono-, Di-, Tri- und Tetraestern von Pentaerythrit mit linearen gesättigten und ungesättigten Fettsäuren mit 12 - 30, insbesondere 14 - 22 Kohlenstoffatomen, sowie Mischungen dieser Strukturanten,
und zusätzlich dazu
g) Wasser in einer Gesamtmenge von 30 - 90 Gew.-%, bevorzugt 40 - 80 Gew.-%, besonders bevorzugt 60 - 78 Gew.-%, außerordentlich bevorzugt 65 - 73 Gew.-%,
auf die Haut, insbesondere die Haut der Achselhöhlen, aufgetragen wird, wobei sich die Gew.-%-Angaben jeweils auf das Gesamtgewicht der Zusammensetzung beziehen.
Bezüglich bevorzugter Ausführungsformen des erfindungsgemäßen Verfahrens gilt mutatis mutandis das zu den erfindungsgemäßen Zusammensetzungen Gesagte.

Ein weiterer Gegenstand der vorliegenden Anmeldung ist die Verwendung einer schweißhemmenden Zusammensetzung in Form einer Öl-in-Wasser-Emulsion, die keine Mikroemulsion ist, enthaltend

a) mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in dem Mittel beziehen, und zusätzlich dazu
b) in einer Gesamtmenge von 0,1 - 2 Gew.-%, bevorzugt 0,3 - 1,5 Gew.-%, besonders bevorzugt 0,5 - 1,1 Gew.-%, außerordentlich bevorzugt 0,6 - 0,8 Gew.-%, mindestens eine oberflächenaktive Verbindung mit einem HLB-Wert im Bereich von 9 bis 15, ausgewählt aus den Partialestern eines Polyglycerins, das 3, 4 oder 5 Glycerin-Einheiten umfasst, mit einer linearen oder verzweigten, gesättigten oder ungesättigten Carbonsäure mit 8 bis 22 C-Atomen und mit einer organischen Genusssäure,

und zusätzlich dazu

c) in einer Gesamtmenge von 0,1 - 2,0 Gew.-%, bevorzugt 0,3 - 1,2 Gew.-%, besonders bevorzugt 0,6 - 1,1 Gew.-%, außerordentlich bevorzugt 0,8 - 1,0 Gew.-%, mindestens ein N-Acyl-L-glutaminsäure-Natriumsalz der Formel (GLUT-1)

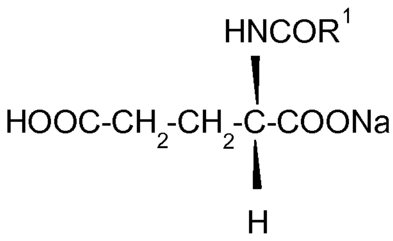

(GLUT-1),

worin $R^1CO$ eine lineare oder verzweigte, gesättigte oder ungesättigte Acylgruppe mit 6 bis 22 C-Atomen, bevorzugt mit 8 bis 18 C-Atomen, darstellt;

und zusätzlich dazu

d) mindestens ein unter Normalbedingungen flüssiges kosmetisches Öl, das kein Riechstoff und kein ätherisches Öl ist, in einer Gesamtmenge von 0,5 - 10 Gew.-%, bevorzugt 1 - 8 Gew.-%, besonders bevorzugt 1,5 - 6 Gew.-%, außerordentlich bevorzugt 2 - 4,5 Gew.-%,

und zusätzlich dazu

e) mindestens ein Hydrogel bildendes Polymer in einer Gesamtmenge von 0,08 - 1 Gew.-%, bevorzugt 0,1 - 0,8 Gew.-%, besonders bevorzugt 0,15 - 0,6 Gew.-%, außerordentlich bevorzugt 0,2 - 0,4 Gew.-%,

und zusätzlich dazu

f) in einer Gesamtmenge von 0,05 bis 4 Gew.-%, bevorzugt 0,5 - 3,5 Gew.-%, besonders bevorzugt 1 - 3 Gew.-% und außerordentlich bevorzugt 1,5 - 2,5 Gew.-%, mindestens einen Strukturanten, ausgewählt aus linearen, gesättigten 1-Alkanolen mit 14 bis 22 C-Atomen, Glycerylmono- und -diestern von linearen oder verzweigten, gesättigten oder ungesättigten Carbonsäuren mit 8 bis 22 C-Atomen, linearen, gesättigten 1-Alkancarbonsäuren mit 14 bis 22 C-Atomen, Mono- und Diestern von Ethylenglycol mit linearen gesättigten und ungesättigten Fettsäuren mit 12 - 30, insbesondere 14 - 22 Kohlenstoffatomen, den Mono-, Di-, Tri- und Tetraestern von Pentaerythrit mit linearen gesättigten und ungesättigten Fettsäuren mit 12 - 30, insbesondere 14 - 22 Kohlenstoffatomen, sowie Mischungen dieser Strukturanten, und zusätzlich dazu

g) Wasser in einer Gesamtmenge von 30 - 90 Gew.-%, bevorzugt 40 - 80 Gew.-%, besonders bevorzugt 60 - 78 Gew.-%, außerordentlich bevorzugt 65 - 73 Gew.-%,

zur nicht-therapeutischen, kosmetischen schweißhemmenden Behandlung des Körpers, wobei sich die Gew.-%-Angaben jeweils auf das Gesamtgewicht der Zusammensetzung beziehen. Bezüglich bevorzugter Ausführungsformen der erfindungsgemäßen Verwendung gilt mutatis mutandis das zu den erfindungsgemäßen Zusammensetzungen Gesagte.

Die folgenden Beispiele sollen den Gegenstand der vorliegenden Erfindung verdeutlichen, ohne ihn hierauf zu beschränken (alle Angaben in Gew.-%).

Antitranspirant-Rollons in Form einer Öl-in-Wasser-Emulsion (keine Mikroemulsionen)

| Handelsname | INCI | Nr. 1 | Nr. 2 | Nr. 3 |
|---|---|---|---|---|
| Eumulgin SG | SODIUM STEAROYL GLUTAMATE | 0,6 | 0,6 | 0,8 |
| Lanette 18 | STEARYL ALCOHOL | 2,5 | 1,5 | 1,5 |
| Myritol 318 | CAPRYLIC/CAPRIC TRIGLYCERIDE | 2,5 | 2,5 | 2 |
| Tego Care PSC 3 | Polyglyceryl-3 Dicitrate/ Stearate | 0,8 | 0,8 | 0,6 |
| Tylose H 100000 YP2 | HYDROXYETHYLCELLULOSE | 0,25 | 0,25 | 0,25 |
| Al-Chlorohydrate 50 % naS | ALUMINUM CHLOROHYDRATE | 40 | 20 | 10 |
| 1,2-Propandiol | PROPYLENE GLYCOL | 5 | 5 | 5 |
| Parfum | PARFUM | 1 | 1 | 1 |
| Wasser VE (vollentsalzt) | AQUA (WATER) | 47,35 | 68,35 | 78,85 |

| Handelsname | INCI | Nr. 4 | Nr. 5 | Nr. 6 |
|---|---|---|---|---|
| Eumulgin SG | SODIUM STEAROYL GLUTAMATE | 0,95 | 1 | 0,4 |
| Lanette 18 | STEARYL ALCOHOL | 1,5 | 1,5 | 1,5 |
| Myritol 318 | CAPRYLIC/CAPRIC TRIGLYCERIDE | 2,8 | 2,5 | 2,5 |
| Tego Care PSC 3 | Polyglyceryl-3 Dicitrate/ Stearate | 0,5 | 0,4 | 1 |
| Tylose H 100000 YP2 | HYDROXYETHYLCELLULOSE | 0,3 | 0,25 | 0,3 |
| Al-Chlorohydrate 50 % naS | ALUMINUM CHLOROHYDRATE | 20 | 10 | 10 |
| 1,2-Propandiol | PROPYLENE GLYCOL | 5 | 5 | 5 |
| Parfum | PARFUM | 1 | 1 | 1 |
| Wasser VE (vollentsalzt) | AQUA (WATER) | 67,9 | 78,35 | 78,3 |

Al-Chlorohydrate 50 % naS: 50 Gew.-%ige wässrige Lösung von nicht aktiviertem Aluminiumchlorhydrat

## Patentansprüche

**1.** Schweißhemmende Zusammensetzung in Form einer Öl-in-Wasser-Emulsion, die keine Mikroemulsion ist, enthaltend

a) mindestens ein schweißhemmendes Aluminiumsalz in einer Gesamtmenge von 2 - 40 Gew.-%, bevorzugt 8 - 35 Gew.-%, besonders bevorzugt 10 - 28 Gew.-% und außerordentlich bevorzugt 12 - 20 Gew.-%, wobei sich die Gew.-%-Angaben auf das Gesamtgewicht der kristallwasserfreien und ligandenfreien Aktivsubstanz (USP) in dem Mittel beziehen,
und zusätzlich dazu
b) in einer Gesamtmenge von 0,1 - 2 Gew.-%, bevorzugt 0,3 - 1,5 Gew.-%, besonders bevorzugt 0,5 - 1,1 Gew.-%, außerordentlich bevorzugt 0,6 - 0,8 Gew.-%, mindestens eine oberflächenaktive Verbindung mit einem HLB-Wert im Bereich von 9 bis 15, ausgewählt aus den Partialestern eines Polyglycerins, das 3, 4 oder 5 Glycerin-Einheiten umfasst, mit einer linearen oder verzweigten, gesättigten oder ungesättigten Carbonsäure mit 8 bis 22 C-Atomen und mit einer organischen Genusssäure,
und zusätzlich dazu
c) in einer Gesamtmenge von 0,1 - 2,0 Gew.-%, bevorzugt 0,3 - 1,2 Gew.-%, besonders bevorzugt 0,6 - 1,1 Gew.-%, außerordentlich bevorzugt 0,8 - 1,0 Gew.-%, mindestens ein N-Acyl-L-glutaminsäure-Natriumsalz der Formel (GLUT-1)

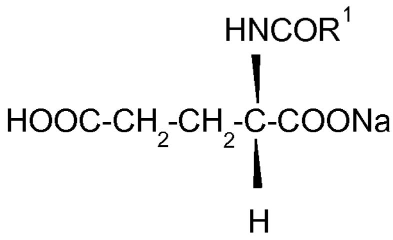

(GLUT-1),

worin $R^1CO$ eine lineare oder verzweigte, gesättigte oder ungesättigte Acylgruppe mit 6 bis 22 C-Atomen, bevorzugt mit 8 bis 18 C-Atomen, darstellt;

und zusätzlich dazu

d) mindestens ein unter Normalbedingungen flüssiges kosmetisches Öl, das kein Riechstoff und kein ätherisches Öl ist, in einer Gesamtmenge von 0,5 - 10 Gew.-%, bevorzugt 1 - 8 Gew.-%, besonders bevorzugt 1,5 - 6 Gew.-%, außerordentlich bevorzugt 2 - 4,5 Gew.-%,

und zusätzlich dazu

e) mindestens ein Hydrogel bildendes Polymer in einer Gesamtmenge von 0,08 - 1 Gew.-%, bevorzugt 0,1 - 0,8 Gew.-%, besonders bevorzugt 0,15 - 0,6 Gew.-%, außerordentlich bevorzugt 0,2 - 0,4 Gew.-%,

und zusätzlich dazu

f) in einer Gesamtmenge von 0,05 bis 4 Gew.-%, bevorzugt 0,5 - 3,5 Gew.-%, besonders bevorzugt 1 - 3 Gew.-% und außerordentlich bevorzugt 1,5 - 2,5 Gew.-%, mindestens einen Strukturanten, ausgewählt aus linearen, gesättigten 1-Alkanolen mit 14 bis 22 C-Atomen, Glycerylmono- und -diestern von linearen oder verzweigten, gesättigten oder ungesättigten Carbonsäuren mit 8 bis 22 C-Atomen, linearen, gesättigten 1-Alkancarbonsäuren mit 14 bis 22 C-Atomen, Mono- und Diestern von Ethylenglycol mit linearen gesättigten und ungesättigten Fettsäuren mit 12 - 30, insbesondere 14 - 22 Kohlenstoffatomen, den Mono-, Di-, Tri- und Tetraestern von Pentaerythrit mit linearen gesättigten und ungesättigten Fettsäuren mit 12 - 30, insbesondere 14 - 22 Kohlenstoffatomen, sowie Mischungen dieser Strukturanten,

und zusätzlich dazu

g) Wasser in einer Gesamtmenge von 30 - 90 Gew.-%, bevorzugt 40 - 80 Gew.-%, besonders bevorzugt 60 - 78 Gew.-%, außerordentlich bevorzugt 65 - 73 Gew.-%,

wobei sich die Gew.-%-Angaben jeweils auf das Gesamtgewicht der Zusammensetzung beziehen.

2. Zusammensetzung nach Anspruch 1, **dadurch gekennzeichnet, dass** Polyethylenglycol-haltige und Polypropylenglycol-haltige Verbindungen in einer Gesamtmenge von 0 bis maximal 0,3 Gew.-%, bevorzugt von 0 bis maximal 0,2 Gew.-%, besonders bevorzugt von 0 bis maximal 0,1 Gew.-%, enthalten sind.

3. Zusammensetzung nach Anspruch 1 oder 2, **dadurch gekennzeichnet, dass** die mindestens eine oberflächenaktive Verbindung b) mit einem HLB-Wert im Bereich von 9 bis 15 ausgewählt ist aus den Partialestern eines Polyglycerins, das 3, 4 oder 5 Glycerin-Einheiten umfasst, mit einer linearen oder verzweigten, gesättigten oder ungesättigten Carbonsäure mit 8 bis 22 C-Atomen und mit Citronensäure.

4. Zusammensetzung nach Anspruch 1, 2 oder 3, **dadurch gekennzeichnet, dass** die mindestens eine oberflächenaktive Verbindung b) mit einem HLB-Wert im Bereich von 9 bis 15 ausgewählt ist aus den Partialestern eines Polyglycerins, das 3, 4 oder 5 Glycerin-Einheiten umfasst, mit Citronensäure und mit einer linearen oder verzweigten, gesättigten oder ungesättigten Carbonsäure mit 8 bis 22 C-Atomen verestert sind, die bevorzugt ausgewählt ist aus 2-Ethylhexansäure, n-Octansäure, n-Decansäure, n-Dodecansäure, n-Tetradecansäure, n-Hexadecansäure (Palmitinsäure), n-Octadecansäure (Stearinsäure), Isostearinsäure, 12-Hydroxystearinsäure, Ölsäure, Linolsäure, Linolensäure, n-Eicosansäure und n-Docosansäure, jeweils mit endständiger Säuregruppe, wobei die Veresterung mit Stearinsäure besonders bevorzugt ist.

5. Zusammensetzung nach Anspruch 1, 2, 3 oder 4, **dadurch gekennzeichnet, dass** die mindestens eine oberflächenaktive Verbindung b) mit einem HLB-Wert im Bereich von 9 bis 15 ausgewählt ist aus den Partialestern eines Polyglycerins, das 3 Glycerin-Einheiten umfasst, mit Citronensäure und mit einer linearen oder verzweigten, gesättigten oder ungesättigten Carbonsäure mit 8 bis 22 C-Atomen, die ausgewählt ist aus 2-Ethylhexansäure, n-Octansäure, n-Decansäure, n-Dodecansäure, n-Tetradecansäure, n-Hexadecansäure (Palmitinsäure), n-Octadecansäure (Stearinsäure), Isostearinsäure, 12-Hydroxystearinsäure, Ölsäure, Linolsäure, Linolensäure, n-Eicosansäure und

n-Docosansäure, jeweils mit endständiger Säuregruppe, wobei die Veresterung mit Stearinsäure besonders bevorzugt ist.

**6.** Zusammensetzung nach Anspruch 1, 2, 3, 4 oder 5, **dadurch gekennzeichnet, dass** die mindestens eine oberflächenaktive Verbindung b) mit einem HLB-Wert im Bereich von 9 bis 15 ausgewählt ist aus den Partialestern eines Polyglycerins, das 3 Glycerin-Einheiten umfasst, mit Citronensäure und mit Stearinsäure, die die INCI-Bezeichnung Polyglyceryl-3 Dicitrate/ Stearate oder Polyglyceryl-3 Stearate/Dicitrate tragen.

**7.** Zusammensetzung nach Anspruch 1, 2, 3, 4, 5 oder 6, **dadurch gekennzeichnet, dass** das mindestens eine N-Acyl-L-glutaminsäure-Natriumsalz der Formel (GLUT-1) ausgewählt ist aus Verbindungen, worin $R^1CO$ eine n-Hexanoyl-, n-Octanoyl-, n-Decanoyl-, n-Lauroyl-, Myristoyl-, Palmitoyl-, Stearoyl-, Arachidoyl-, Behenoyl- oder Cocoyl-Gruppe, bevorzugt eine Stearoyl-Gruppe, darstellt, jeweils mit endständiger Carbonylgruppe.

**8.** Zusammensetzung nach Anspruch 1, 2, 3, 4, 5, 6 oder 7, **gekennzeichnet durch** ein Gewichtsverhältnis von Gesamtgewicht an Polyglycerinester b) zu Gesamtgewicht an N-Acyl-L-glutaminsäure-Natriumsalz der Formel (GLUT-1) im Bereich von 0,5 bis 1,0, bevorzugt 0,6 bis 0,8.

**9.** Zusammensetzung nach Anspruch 1, 2, 3, 4, 5, 6, 7 oder 8, **dadurch gekennzeichnet, dass** das mindestens eine ein Hydrogel bildende Polymer e) ausgewählt ist aus Celluloseethern, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose, Methylhydroxyethylcellulose, weiterhin Xanthan-Gum, Sclerotium Gum, Succinoglucanen, Polygalactomannanen, insbesondere Guar-Gums und Johannisbrotkernmehl (Locust Bean Gum), insbesondere Guar-Gum und Locust Bean Gum selbst und den nichtionischen Hydroxyalkylguarderivaten und Johannisbrotkernmehl-Derivaten, wie Hydroxypropylguar, Carboxymethylhydroxypropylguar, Hydroxypropylmethylguar, Hydroxyethylguar und Carboxymethylguar, weiterhin Pectinen, Agar, Carragheen (Carrageenan), Traganth, Gummi arabicum, Karayagummi, Taragummi, Gellan, Gelatine, Casein, Propylenglycolalginat, Alginsäuren und deren Salze, insbesondere Natriumalginat, Kaliumalginat und Calciumalginat, weiterhin Polyvinylpyrrolidonen, Polyvinylalkoholen, Polyacrylamiden, weiterhin physikalisch (z. B. durch Vorverkleisterung) und/oder chemisch modifizierten Stärken, insbesondere hydroxypropylierten Stärkephosphaten und Octenylstärkesuccinaten und deren Aluminium-, Calcium- oder Natriumsalzen, sowie Mischungen hiervon, bevorzugt ausgewählt aus Celluloseethern, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose und Methylhydroxyethylcellulose, sowie Mischungen hiervon; bevorzugt ausgewählt aus Celluloseethern, besonders bevorzugt aus Hydroxyalkylcellulosen, insbesondere aus Hydroxypropylcellulose, Hydroxypropylmethylcellulose, Hydroxyethylcellulose, Carboxymethylcellulose, Cetylhydroxyethylcellulose, Hydroxybutylmethylcellulose und Methylhydroxyethylcellulose, sowie Mischungen hiervon, außerordentlich bevorzugt ausgewählt aus Hydroxyethylcellulose.

**10.** Zusammensetzung nach Anspruch 1, 2, 3, 4, 5, 6, 7, 8 oder 9, **dadurch gekennzeichnet, dass** der mindestens eine Strukturant ausgewählt ist aus Myristylalkohol, Cetylalkohol, Stearylalkohol, Arachidylalkohol, Behenylalkohol, Glycerylmonostearat, Glyceryldistearat, Glycerylmonocaprinat, Glyceryldicaprinat, Glycerylmonocaprylat, Glyceryldicaprylat, Glycerylmonolaurat, Glyceryldilaurat, Glycerylmonomyristat, Glyceryldimyristat, Glycerylmonopalmitat, Glyceryldipalmitat, Glycerylmono-12-hydroxystearat, Glyceryldi-12-hydroxystearat, Glycerylmonooleat, Glyceryldioleat, Glycerylmonolanolat, Glyceryldilanolat, sowie Mischungen dieser Substanzen.

**11.** Zusammensetzung nach Anspruch 1, 2, 3, 4, 5, 6, 7, 8, 9 oder 10, **gekennzeichnet durch** eine dynamische Viskosität im Bereich von 300 - 10000 mPas, bevorzugt 800 - 7500 mPas, besonders bevorzugt 1000 - 5000 mPas, gemessen mit einem Brookfield-Viskosimeter, Spindel RV 4, 20 $s^{-1}$, ohne Helipath, bei 20 °C Umgebungstemperatur und 20 °C Probentemperatur.

**12.** Zusammensetzung nach Anspruch 1, 2, 3, 4, 5, 6, 7, 8, 9, 10 oder 11, enthaltend

a) 2 - 40 Gew.-% schweißhemmendes Aluminiumsalz,
b) 0,1 - 2 Gew.-% Polyglyceryl-3 Dicitrate/Stearate,
c) 0,1 -2,0 Gew.-% N-Stearoylnatrium-L-glutamat,
d) 0,5 - 10 Gew.-% kosmetisches Öl,
e) 0,08 - 1 Gew.-% Hydroxyethylcellulose,
f) 0,05 - 4 Gew.-% Stearylalkohol und

g) 30 - 90 Gew.-% Wasser.

**13.** Zusammensetzung nach Anspruch 1, 2, 3, 4, 5, 6, 7, 8, 9, 10, 11 oder 12, enthaltend

a) 8 - 35 Gew.-% schweißhemmendes Aluminiumsalz,
b) 0,3 - 1,5 Gew.-% Polyglyceryl-3 Dicitrate/Stearate,
c) 0,3 - 1,2 Gew.-% N-Stearoylnatrium-L-glutamat,
d) 1 - 8 Gew.-% kosmetisches Öl,
e) 0,1 -0,8 Gew.-% Hydroxyethylcellulose,
f) 0,5 - 3,5 Gew.-% Stearylalkohol und
g) 40 - 80 Gew.-% Wasser.

**14.** Zusammensetzung nach Anspruch 1, 2, 3, 4, 5, 6, 7, 8, 9, 10, 11, 12 oder 13, enthaltend

a) 10 - 28 Gew.-% schweißhemmendes Aluminiumsalz,
b) 0,5 - 1,1 Gew.-% Polyglyceryl-3 Dicitrate/Stearate,
c) 0,6 - 1,1 Gew.-% N-Stearoylnatrium-L-glutamat,
d) 1,5 - 6 Gew.-% kosmetisches Öl,
e) 0,15 - 0,6 Gew.-% Hydroxyethylcellulose,
f) 1 - 3 Gew.-% Stearylalkohol und
g) 60 - 78 Gew.-% Wasser.

**15.** Zusammensetzung nach Anspruch 1, 2, 3, 4, 5, 6, 7, 8, 9, 10, 11, 12, 13 oder 14, enthaltend

a) 12 - 20 Gew.-% schweißhemmendes Aluminiumsalz,
b) 0,6 - 0,8 Gew.-% Polyglyceryl-3 Dicitrate/Stearate,
c) 0,8 - 1,0 Gew.-% N-Stearoylnatrium-L-glutamat,
d) 2 - 4,5 Gew.-% kosmetisches Öl,
e) 0,2 - 0,4 Gew.-% Hydroxyethylcellulose,
f) 1,5 - 2,5 Gew.-% Stearylalkohol und
g) 65 - 73 Gew.-% Wasser.

## Claims

**1.** A perspiration-inhibiting composition in the form of an oil-in-water emulsion that is not a microemulsion, containing

a) at least one perspiration-inhibiting aluminum salt in a total quantity from 2 to 40 wt%, preferably 8 to 35 wt%, particularly preferably 10 to 28 wt%, and extraordinarily preferably 12 to 20 wt%, wherein the "wt%" indications refer to the total weight of active substance (USP), free of water of crystallization and free of ligands, in the agent, and in addition thereto
b) in a total quantity from 0.1 to 2 wt%, preferably 0.3 to 1.5 wt%, particularly preferably 0.5 to 1.1 wt%, extraordinarily preferably 0.6 to 0.8 wt%, at least one surface-active compound having an HLB value in the range from 9 to 15, selected from the partial esters of a polyglycerol that comprises 3, 4, or 5 glycerol units with a linear or branched, saturated or unsaturated carboxylic acid having 8 to 22 carbon atoms and with an organic edible acid, and in addition thereto
c) in a total quantity from 0.1 to 2.0 wt%, preferably 0.3 to 1.2 wt%, particularly preferably 0.6 to 1.1 wt%, extraordinarily preferably 0.8 to 1.0 wt%, at least one N-acyl-L-glutamic acid sodium salt of formula (GLUT-1)

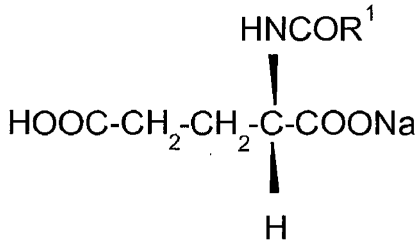

(GLUT-1),

in which $R^1CO$ represents a linear or branched, saturated or unsaturated acyl group having 6 to 22 carbon atoms, preferably having 8 to 18 carbon atoms,
and in addition thereto
d) at least one cosmetic oil, liquid under standard conditions, that is not a fragrance and not an essential oil, in a total quantity from 0.5 to 10 wt%, preferably 1 to 8 wt%, particularly preferably 1.5 to 6 wt%, extraordinarily preferably 2 to 4.5 wt%,
and in addition thereto
e) at least one hydrogel-forming polymer in a total quantity from 0.08 to 1 wt%, preferably 0.1 to 0.8 wt%, particularly preferably 0.15 to 0.6 wt%, extraordinarily preferably 0.2 to 0.4 wt%,
and in addition thereto
f) in a total quantity from 0.05 to 4 wt%, preferably 0.5 to 3.5 wt%, particularly preferably 1 to 3 wt%, and extraordinarily preferably 1.5 to 2.5 wt%, at least one structuring agent selected from linear saturated 1-alkanols having 14 to 22 carbon atoms, glyceryl mono- and diesters of linear or branched, saturated or unsaturated carboxylic acids having 8 to 22 carbon atoms, linear saturated 1-alkanecarboxylic acids having 14 to 22 carbon atoms, mono- and diesters of ethylene glycol with linear saturated and unsaturated fatty acids having 12 to 30, in particular 14 to 22 carbon atoms, the mono-, di-, tri-, and tetraesters of pentaerythritol with linear saturated and unsaturated fatty acids having 12 to 30, in particular 14 to 22 carbon atoms, as well as mixtures of said structuring agents,
and in addition thereto
g) water in a total quantity from 30 to 90 wt%, preferably 40 to 80 wt%, particularly preferably 60 to 78 wt%, extraordinarily preferably 65 to 73 wt%,

wherein the "wt%" indications refer in each case to the total weight of the composition.

**2.** The composition according to Claim 1, **characterized in that** polyethylene-glycol-containing and polypropylene-glycol-containing compounds are contained in a total quantity from 0 to a maximum of 0.3 wt%, preferably from 0 to a maximum of 0.2 wt%, particularly preferably from 0 to a maximum of 0.1 wt%.

**3.** The composition according to Claim 1 or 2, **characterized in that** the at least one surface-active compound b) having an HLB value in the range from 9 to 15 is selected from partial esters of a polyglycerol that comprises 3, 4, or 5 glycerol units with a linear or branched, saturated or unsaturated carboxylic acid having 8 to 22 carbon atoms, and with citric acid.

**4.** The composition according to Claim 1, 2, or 3, **characterized in that** the at least one surface-active compound b) having an HLB value in the range from 9 to 15 is selected from partial esters of a polyglycerol that comprises 3, 4, or 5 glycerol units which are esterified with citric acid and with a linear or branched, saturated or unsaturated carboxylic acid having 8 to 22 carbon atoms that is preferably selected from 2-ethylhexanoic acid, n-octanoic acid, n-decanoic acid, n-dodecanoic acid, n-tetradecanoic acid, n-hexadecanoic acid (palmitic acid), n-octadecanoic acid (stearic acid), isostearic acid, 12-hydroxystearic acid, oleic acid, linoleic acid, linolenic acid, n-eicosanoic acid, and n-docosanoic acid, in each case having a terminal acid group, wherein esterification with stearic acid is particularly preferred.

**5.** The composition according to Claim 1, 2, 3, or 4, **characterized in that** the at least one surface-active compound b) having an HLB value in the range from 9 to 15 is selected from partial esters of a polyglycerol that comprises 3 glycerol units with citric acid and with a linear or branched, saturated or unsaturated carboxylic acid having 8 to 22 carbon atoms, that is selected from 2-ethylhexanoic acid, n-octanoic acid, n-decanoic acid, n-dodecanoic acid, n-tetradecanoic acid, n-hexadecanoic acid (palmitic acid), n-octadecanoic acid (stearic acid), isostearic acid, 12-hydroxystearic acid, oleic acid, linoleic acid, linolenic acid, n-eicosanoic acid, and n-docosanoic acid, in each case having a terminal acid group, wherein esterification with stearic acid is particularly preferred.

**6.** The composition according to Claim 1, 2, 3, 4, or 5, **characterized in that** the at least one surface-active compound b) having an HLB value in the range from 9 to 15 is selected from partial esters of a polyglycerol that comprises 3 glycerol units with citric acid and with stearic acid, which carry the INCI name Polyglyceryl-3 Dicitrate/Stearate or Polyglyceryl-3 Stearate/Dicitrate.

**7.** The composition according to Claim 1, 2, 3, 4, 5, or 6, **characterized in that** the at least one N-acyl-L-glutamic acid sodium salt of formula (GLUT-1) is selected from compounds in which $R^1CO$ represents an n-hexanoyl, n-octanoyl, n-decanoyl, n-lauroyl, myristoyl, palmitoyl, stearoyl, arachidoyl, behenoyl, or cocoyl group, preferably a stearoyl

group, in each case having a terminal carbonyl group.

**8.** The composition according to Claim 1, 2, 3, 4, 5, 6, or 7, **characterized by** a weight ratio of the total weight of polyglycerol ester b) to the total weight of N-acyl-L-glutamic acid sodium salt of formula (GLUT-1) in the range from 0.5 to 1.0, preferably 0.6 to 0.8.

**9.** The composition according to Claim 1, 2, 3, 4, 5, 6, 7, or 8, **characterized in that** the at least one hydrogel-forming polymer e) is selected from cellulose ethers, especially hydroxyalkyl celluloses, in particular hydroxypropyl cellulose, hydroxypropylmethyl cellulose, hydroxyethyl cellulose, carboxymethyl cellulose, cetylhydroxyethyl cellulose, hydroxybutylmethyl cellulose, methylhydroxyethyl cellulose, furthermore xanthan gum, sclerotium gum, succinoglucans, polygalactomannans, in particular guar gums und locust bean gum, in particular guar gum und locust bean gum itself and the nonionic hydroxyalkyl guar derivatives and locust bean gum derivatives such as hydroxypropyl guar, carboxymethylhydroxypropyl guar, hydroxypropylmethyl guar, hydroxyethyl guar, and carboxymethyl guar, furthermore pectins, agar, carrageenan, tragacanth, gum arabic, karaya gum, tara gum, gellan, gelatin, casein, propylene glycol alginate, alginic acids and salts thereof, in particular sodium alginate, potassium alginate, and calcium alginate, furthermore polyvinylpyrrolidones, polyvinyl alcohols, polyacrylamides, furthermore starches modified physically (e.g. by pregelatinization) and/or chemically, in particular hydroxypropylated starch phosphates and octenyl starch succinates and aluminum, calcium, or sodium salts thereof, as well as mixtures thereof, preferably selected from cellulose ethers, especially hydroxyalkyl celluloses, in particular hydroxypropyl cellulose, hydroxypropylmethyl cellulose, hydroxyethyl cellulose, carboxymethyl cellulose, cetylhydroxyethyl cellulose, hydroxybutylmethyl cellulose, and methylhydroxyethyl cellulose, as well as mixtures thereof; preferably selected from cellulose ethers, particularly preferably from hydroxyalkyl celluloses, in particular from hydroxypropyl cellulose, hydroxypropylmethyl cellulose, hydroxyethyl cellulose, carboxymethyl cellulose, cetylhydroxyethyl cellulose, hydroxybutylmethyl cellulose und methylhydroxyethyl cellulose, as well as mixtures thereof, extraordinarily preferably selected from hydroxyethyl cellulose.

**10.** The composition according to Claim 1, 2, 3, 4, 5, 6, 7, 8, or 9, **characterized in that** the at least one structuring agent is selected from myristyl alcohol, cetyl alcohol, stearyl alcohol, arachidyl alcohol, behenyl alcohol, glyceryl monostearate, glyceryl distearate, glyceryl monocaprinate, glyceryl dicaprinate, glyceryl monocaprylate, glyceryl dicaprylate, glyceryl monolaurate, glyceryl dilaurate, glyceryl monomyristate, glyceryl dimyristate, glyceryl monopalmitate, glyceryl dipalmitate, glyceryl mono-12-hydroxystearate, glyceryl di-12-hydroxystearate, glyceryl monooleate, glyceryl dioleate, glyceryl monolanolate, glyceryl dilanolate, and mixtures of these substances.

**11.** The composition according to Claim 1, 2, 3, 4, 5, 6, 7, 8, 9, or 10, **characterized by** a dynamic viscosity in the range from 300 to 10,000 mPas, preferably 800 to 7500 mPas, particularly preferably 1000 to 5000 mPas, measured with a Brookfield viscometer, spindle RV 4, 20 $s^{-1}$, no Helipath, at a 20°C ambient temperature and 20°C sample temperature.

**12.** The composition according to Claim 1, 2, 3, 4, 5, 6, 7, 8, 9, 10, or 11, containing

a) 2 to 40 wt% perspiration-inhibiting aluminum salt,
b) 0.1 to 2 wt% Polyglyceryl-3 Dicitrate/Stearate,
c) 0.1 to 2.0 wt% N-stearoylsodium-L-glutamate,
d) 0.5 to 10 wt% cosmetic oil,
e) 0.08 to 1 wt% hydroxyethyl cellulose,
f) 0.05 to 4 wt% stearyl alcohol, and
g) 30 to 90 wt% water.

**13.** The composition according to Claim 1, 2, 3, 4, 5, 6, 7, 8, 9, 10, 11, or 12, containing

a) 8 to 35 wt% perspiration-inhibiting aluminum salt,
b) 0.3 to 1.5 wt% Polyglyceryl-3 Dicitrate/Stearate,
c) 0.3 to 1.2 wt% N-stearoylsodium-L-glutamate,
d) 1 to 8 wt% cosmetic oil,
e) 0.1 to 0.8 wt% hydroxyethyl cellulose,
f) 0.5 to 3.5 wt% stearyl alcohol, and
g) 40 to 80 wt% water.

**14.** The composition according to Claim 1, 2, 3, 4, 5, 6, 7, 8, 9, 10, 11, 12, or 13, containing

a) 10 to 28 wt% perspiration-inhibiting aluminum salt,
b) 0.5 to 1.1 wt% Polyglyceryl-3 Dicitrate/Stearate,
c) 0.6 to 1.1 wt% N-stearoylsodium-L-glutamate,
d) 1.5 to 6 wt% cosmetic oil,
e) 0.15 to 0.6 wt% hydroxyethyl cellulose,
f) 1 to 3 wt% stearyl alcohol, and
g) 60 to 78 wt% water.

**15.** The composition according to Claim 1, 2, 3, 4, 5, 6, 7, 8, 9, 10, 11, 12, 13, or 14, containing

a) 12 to 20 wt% perspiration-inhibiting aluminum salt,
b) 0.6 to 0.8 wt% Polyglyceryl-3 Dicitrate/Stearate,
c) 0.8 to 1.0 wt% N-stearoylsodium-L-glutamate,
d) 2 to 4.5 wt% cosmetic oil,
e) 0.2 to 0.4 wt% hydroxyethyl cellulose,
f) 1.5 to 2.5 wt% stearyl alcohol, and
g) 65 to 73 wt% water.

## Revendications

**1.** Composition anti-transpirante se présentant sous la forme d'une émulsion huile-dans-eau qui n'est pas une microémulsion, ladite composition anti-transpirante contenant

a) au moins un sel d'aluminium anti-transpiraht dans une quantité totale de 2 à 40% en poids, de préférence de 8 à 35% en poids, de manière particulièrement préférée de 10 à 28% en poids et de manière extraordinairement préférée de 12 à 20% en poids, les données en % en poids se rapportant au poids total de la substance active sans eau de cristallisation ni ligand (USP) dans l'agent,
et en outre,
b) dans une quantité totale de 0,1 à 2% en poids, de préférence de 0,3 à 1,5% en poids, de manière particulièrement préférée de 0,5 à 1,1% en poids, de manière extraordinairement préférée de 0,6 à 0,8% en poids, au moins un composé tensio-actif ayant une valeur HLB dans la gamme de 9 à 15, choisi à partir des esters partiels d'un polyglycérol qui comporte 3, 4 ou 5 unités glycérol, avec un acide carboxylique linéaire ou ramifié, saturé ou insaturé, ayant 8 à 22 atomes de carbone et un acide organique comestible,
et en outre,
c) dans une quantité totale de 0,1 à 2,0% en poids, de préférence de 0,3 à 1,2% en poids, de manière particulièrement préférée de 0,6 à 1,1% en poids, de manière extraordinairement préférée de 0,8 à 1,0% en poids, au moins un sel de sodium de l'acide N-acyl-L-glutamique de la formule (GLUT-1)

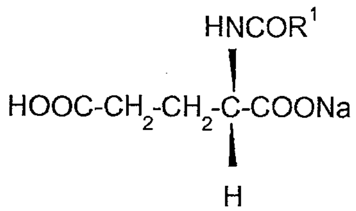

(GLUT-1),

dans laquelle $R^1CO$ est un groupe acyle linéaire ou ramifié, saturé ou insaturé, ayant 6 à 22 atomes de carbone, de préférence 8 à 18 atomes de carbone ;
et en outre
d) au moins une huile cosmétique, liquide dans des conditions normales, qui n'est pas une substance odorante ni une huile essentielle, dans une quantité totale de 0,5 à 10% en poids, de préférence de 1 à 8% en poids, de manière particulièrement préférée de 1,5 à 6% en poids, de manière extraordinairement préférée de 2 à 4,5% en poids,

et en outre
e) au moins un polymère formant un hydrogel dans une quantité totale de 0,08 à 1% en poids, de préférence de 0,1 à 0,8% en poids, de manière particulièrement préférée de 0,15 à 0,6% en poids, de manière extraordinairement préférée de 0,2 à 0,4% en poids,
et en outre,
f) dans une quantité totale de 0,05 à 4% en poids, de préférence de 0,5 à 3,5% en poids, de manière particulièrement préférée de 1 à 3% en poids, et de manière extraordinairement préférée de 1,5 à 2,5% en poids, au moins un structurant, choisi parmi les 1-alcanols linéaires, saturés ayant 14 à 22 atomes de carbone, les mono- et di-esters de glycérol des acides carboxyliques linéaires ou ramifiés, saturés ou insaturés, ayant de 8 à 22 atomes de carbone, les acides 1-alcane-carboxyliques linéaires, saturés, ayant 14 à 22 atomes de carbone, les mono- et diesters d'éthylène-glycol d'acides gras linéaire, saturés et non saturés ayant 12 à 30, en particulier 14 à 22 atomes de carbone, les mono-, di-, tri- et tétra-esters de pentaérythrite avec des acides gras linéaires, saturés et insaturés ayant 12 à 30, en particulier 14 à 22, atomes de carbone, et des mélanges de ces structurants,
et en outre,
g) de l'eau dans une quantité totale de 30 à 90% en poids, de préférence de 40 à 80% en poids, de manière particulièrement préférée de 60 à 78% en poids, de manière extraordinairement préférée de 65 à 73% en poids,

les données en % en poids se rapport à chaque fois au poids total de la composition.

2. Composition selon la revendication 1, **caractérisée en ce que** les composés contenant du polyéthylène-glycol et du polypropylène-glycol sont contenus dans une quantité totale de 0 à 0,3% en poids maximum, de préférence de 0 à 0,2% en poids maximum, de manière particulièrement préférée de 0 à 0,1% en poids maximum.

3. Composition selon la revendication 1 ou 2, **caractérisé en ce que** l'au moins un composé tensio-actif b) ayant une valeur HLB dans la gamme de 9 à 15 est choisi parmi les esters partiels d'un polyglycérol qui comporte 3, 4 ou 5 unités glycérol, avec un acide carboxylique linéaire ou ramifié, saturé ou insaturé, ayant 8 à 22 atomes de carbone et avec de l'acide citrique.

4. Composition selon la revendication 1, 2 ou 3, **caractérisée en ce que** l'au moins un composé tensio-actif b) ayant une valeur HLB dans la gamme de 9 à 15 est choisi parmi les esters partiels d'un polyglycérol comportant 3, 4 ou 5 unités glycérol, qui sont estérifiés avec l'acide citrique et un acide carboxylique linéaire ou ramifié, saturé ou insaturé ayant 8 à 22 atomes de carbone qui est de préférence choisi parmi l'acide 2-éthylhexanoïque, l'acide n-octanoïque, l'acide n-décanoïque, l'acide n-dodécanoïque, l'acide n-tétradécanoïque, l'acide n-hexadécanoïque (acide palmitique), l'acide n-octadécanoïque (acide stéarique), l'acide isostéarique, l'acide 12-hydroxystéarique, l'acide oléique, l'acide linoléique, l'acide linolénique, l'acide n-eicosanoïque et l'acide n-docosanoïque, ayant chacun un groupe acide terminal, l'estérification avec l'acide stéarique étant particulièrement préféré.

5. Composition selon la revendication 1, 2, 3 ou 4, **caractérisée en ce que** l'au moins un composé tensio-actif b) ayant une valeur HLB dans la gamme de 9 à 15 est choisi parmi les esters partiels d'un polyglycérol comportant 3 unités glycérol, avec l'acide citrique et un acide carboxylique linéaire ou ramifié, saturé ou insaturé, ayant 8 à 22 atomes de carbone, qui est choisi parmi l'acide 2-éthylhexanoïque, l'acide n-octanoïque, l'acide n-décanoïque, l'acide n-dodécanoïque, l'acide n-tétradécanoïque, l'acide n-hexadécanoïque (acide palmitique), l'acide n-octadécanoïque (acide stéarique), l'acide isostéarique, l'acide 12-hydroxystéarique, l'acide oléique, l'acide linoléique, l'acide linolénique, l'acide n-eicosanoïque et l'acide n-docosanoïque, ayant chacun un groupe acide terminal, l'estérification avec de l'acide stéarique étant particulièrement préféré.

6. Composition selon la revendication 1, 2, 3, 4 ou 5, **caractérisé en ce que** l'au moins un composé tensio-actif b) ayant une valeur HLB dans la gamme de 9 à 15 est choisi parmi les esters partiels d'un polyglycérol comportant 3 unités glycérol, avec de l'acide citrique et de l'acide stéarique qui ont les désignations INCI dicitrate/stéarate de polyglycérol-3 ou stéarate/dicitrate polyglycérol-3.

7. Composition selon la revendication 1, 2, 3, 4, 5 ou 6, **caractérisé en ce que** l'au moins un sel de sodium de l'acide N-acyl-L-glutamique de la formule (GLUT-1) est choisi parmi les composés dans lesquels $R^1CO$ représente un groupe n-hexanoyle, n-octanoyle, n-décanoyle, n-lauroyle, myristoyle, palmitoyle, stéaroyle, arachidoyle, béhénoyle ou cocoyle, de préférence un groupe stéaroyle, ayant chacun un groupe carbonyle terminal.

8. Composition selon la revendication 1, 2, 3, 4, 5, 6 ou 7, **caractérisé par** un rapport en poids du poids total des esters de polyglycérol b) au poids total de sel de sodium de l'acide N-acyl-L-glutamique de la formule (GLUT-1)

dans la gamme de 0,5 à 1,0, de préférence de 0,6 à 0,8.

**9.** Composition selon la revendication 1, 2, 3, 4, 5, 6, 7 ou 8, **caractérisée en ce que** l'au moins polymère e) formant un hydrogel est choisi parmi lès éthers de cellulose, en particulier les hydroxyalkylcelluloses, en particulier l'hydroxypropylcellulose, l'hydroxypropylméthylcellulose, l'hydroxyéthylcellulose, la carboxyméthylcellulose, la cétylhydroxyéthylcellulose, l'hydroxybutylméthylcellulose, la méthylhydroxyéthylcellulose, en outre la gomme de xanthane, la gomme de sclérotium, les succinoglucanes, les polygalactomannanes, en particulier la gomme de guar et la farine de caroube (Locust Bean Gum), en particulier la gomme de guar et la gomme de caroube eux-mêmes et les dérivés de l'hydroxyalkylguar non ioniques et les dérivés de la farine de caroube, tels que l'hydroxypropylguar, le carboxyméthylhydroxypropylguar, l'hydroxypropylméthylguar, l'hydroxyéthylguar et le carboxyméthylguar, en outre les pectines, l'agar, le carraghénane (Carrageenan), la gomme adragante, la gomme arabique, la gomme de Karaya, la gomme tara, la gellane, la gélatine, la caséine, l'alginate de propylène-glycol, les acides alginiques et leurs sels, en particulier l'alginate de sodium, l'alginate de potassium et l'alginate de calcium, en outre les polyvinylpyrrolidones, les alcools polyvinyliques, les polyacrylamides, en outre les amidons modifiés physiquement (par exemple par prégélatinisation) et/ou chimiquement, en particulier les octényl-succinates d'amidon et les phosphates d'amidon hydroxypropylés et leurs sels d'aluminium-, de calcium ou de sodium, ainsi que leurs mélanges, de préférence choisis parmi les éthers de cellulose, surtout les hydroxyalkylcelluloses, en particulier l'hydroxypropylcellulose, l'hydroxyéthylcellulose, la carboxyméthylcellulose, la cétylhydroxyéthylcellulose, l'hydroxybutylméthylcellulose et la méthylhydroxyéthylcellulose et leurs mélanges ; de préférence choisis parmi les éthers de cellulose, de manière particulièrement préférée parmi les hydroxyalkylcelluloses, en particulier l'hydroxypropylcellulose, l'hydroxypropylméthylcellulose, l'hydroxyéthylcellulose, la carboxyméthylcellulose, la cétylhydroxyéthylcellulose, l'hydroxybutylméthylcellulose et la méthylhydroxyéthylcellulose et leurs mélanges, de manière extraordinairement préférée l'hydroxyéthylcellulose.

**10.** Composition selon la revendication 1, 2, 3, 4, 5, 6, 7, 8 ou 9, **caractérisée en ce que** l'au moins un structurant est choisi parmi l'alcool myristylique, l'alcool cétylique, l'alcool stéarylique, l'alcool arachidylique, l'alcool béhénylique, le monostéarate de glycéryle, le distéarate de glycéryle, le mono-caprate de glycéryle, le dicaprinate de glycéryle, le monocaprylate de glycéryle, le dicaprylate de glycéryle, le monolaurate de glycéryle, le dilaurate de glycéryle, le monomyristate de glycéryle, le dimyristate de glycéryle, le monopalmitate de glycéryle, le dipalmitate de glycéryle, le mono-12-hydroxystéarate de glycéryle, le di-12-hydroxystéarate de glycéryle, le monooléate de glycéryle, le dioléate de glycéryle, le monolanolate de glycéryle, le dilanolate de glycéryle, et des mélanges de ces substances.

**11.** Composition selon la revendication 1, 2, 3, 4, 5, 6, 7, 8, 9 ou 10, **caractérisée par** une viscosité dynamique dans gamme de 300 à 10000 mPas, de préférence de 800 à 7500 mPas, plus préférablement de 1000 à 5000 mPas, mesurée par un viscosimètre Brookfield Spindel RV 4, 20 $s^{-1}$, sans Helipath à une température ambiante de 20°C et une température d'échantillon de 20°C.

**12.** Composition selon la revendication 1, 2, 3, 4, 5, 6, 7, 8, 9, 10 ou 11, comprenant

a) 2 à 40% en poids de sel d'aluminium anti-transpirant,
b) 0,1 à 2% en poids de dicitrates/stéarates de polyglycéryl-3,
c) 0,1 à 2,0% en poids de Sodium-N-stéaroyl-L-glutamate,
d) 0,5 à 10% en poids d'huile cosmétique,
e) 0,08 à 1% en poids d'hydroxyéthylcellulose,
f) 0,05 à 4% en poids d'alcool stéarylique et
g) 30 à 90% en poids d'eau.

**13.** Composition selon la revendication 1, 2, 3, 4, 5, 6, 7, 8, 9, 10, 11 ou 12, comprenant

a) 8 à 35% en poids de sel d'aluminium anti-transpirant,
b) 0,3 à 1,5% en poids de dicitrates/stéarates de polyglycéryl-3,
c) 0,3 à 1,2% en poids de Sodium-N-stéaroyl-L-glutamate,
d) 1 à 8% en poids d'huile cosmétique,
e) 0,1 à 8% en poids d'hydroxyéthylcellulose,
f) 0,5 à 3,5% en poids d'alcool stéarylique et
g) 40 à 80% en poids d'eau.

**14.** Composition selon la revendication 1, 2, 3, 4, 5, 6, 7, 8, 9, 10, 11, 12 ou 13, comprenant

a) 10 à 28% en poids de sel d'aluminium anti-transpirant,
b) 0,5 à 1,1% en poids de dicitrates/stéarates de polyglycéryl-3,
c) 0,6 à 1,1% en poids de Sodium-N-stéaroyl-L-glutamate,
d) 1,5 à 6% en poids d'huile cosmétique,
e) 0,15 à 0,6% en poids d'hydroxyéthylcellulose,
f) 1 à 3% en poids d'alcool stéarylique et
g) 60 à 78% en poids d'eau.

**15.** Composition selon la revendication 1, 2, 3, 4, 5, 6, 7, 8, 9, 10, 11, 12, 13 ou 14, comprenant

a) 12 à 20% en poids de sel d'aluminium anti-transpirant,
b) 0,6 à 0,8% en poids de dicitrates/stéarates de polyglycéryl-3,
c) 0,8 à 1,0% en poids de Sodium-N-stéaroyl-L-glutamate,
d) 2 à 4,5% en poids d'huile cosmétique,
e) 0,2 à 0,4% en poids d'hydroxyéthylcellulose,
f) 1,5 à 2,5% en poids d'alcool stéarylique et
g) 65 à 73% en poids d'eau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 3887692 A
- US 3904741 A
- US 4359456 A
- GB 2048229 A
- GB 1347950 A
- US 4775528 A
- US 6010688 A
- US 2571030 A
- US 4017599 A
- US 7105691 B
- US 5643559 A
- US 5676937 A
- WO 2001099376 A2
- EP 1430879 A1
- DE 10216368 A1
- WO 2003039505 A2
- EP 1428520 A2
- DE 3018132 A1
- GB 2335596 A1
- EP 650720 A1
- EP 1428519 A2
- EP 495918 B1
- WO 2006079934 A
- DE 102010000746 A1
- WO 2010031657 A1
- WO 2010046291 A1